# EUROPEAN PATENT APPLICATION

(11) **EP 2 633 853 A1**
(43) Date of publication of application: **04.09.2013**
(21) Application number: 13169417.6
(22) Date of filing: 12.01.2005
(51) Int. Cl.: A61K 9/22, A61K 31/445, A61K 31/519, A61P 25/18

(54) **Long-term delivery formulations and methods of use thereof**

(30) Priority: 12.01.2004 US 535908 P; 06.10.2004 US 616322 P
(62) Divisional of application: 05705510.5
(71) Applicant: The Trustees of The University of Pennsylvania, Philadelphia, Pennsylvania 19104-6283 (US)
(72) Inventor: Siegel, Steven, Berwyn, PA 19312 (US); Winey, Karen, Philadelphia, PA 19103 (US)
(74) Representative: Pearl Cohen Zedek Latzer UK LLP

(57) **Abstract**

The present invention provides a method, a kit and compositions for long-term release of a drug at a constant therapeutically effective level for nervous system disorders where adherence to therapeutic regimen is problematic. In particular, to the therapy of psychotic disorders.

## Description

### FIELD OF INVENTION

This invention provides long-term delivery formulations comprising a biodegradable matrix and an agent of interest, methods of producing the same, and methods of use thereof. The invention also relates to long-term formulations of Risperidone and 9-OH-Risperidone compositions for the treatment of psychotic disorders.

### BACKGROUND OF THE INVENTION

Psychotic disorders are pathological conditions of the brain characterized by identifiable symptoms that result in abnormalities in cognition, emotion or mood, or in particular, in terms of ones ability to work, interact and be intimate with other people. These disorders may vary in severity of symptoms, duration, and functional impairment. Psychiatric disorders afflict millions of people of all ages worldwide, for extensive periods of time, resulting in tremendous human suffering and economic burden due to lost productivity. In some cases the psychiatric disorder may be acute, lasting only for several weeks to months. In other instances the disorder is chronic, lasting for years or even decades.

The use of pharmacological agents to treat psychotic disorders has greatly increased due to research advances in both neuroscience and molecular biology. In addition, advances in the field resulted in production of new drugs and delivery mechanisms, which are more effective in treating psychosis and other pathological conditions of the brain, and yet have fewer accompanying side effects.

Medication non-compliance is a pervasive problem with many forms of illness. Non-compliance causes particular medical and social problems when the drugs concerned are neuroleptics (anti-psychotics) such as chlorpromazine or haloperidol (Rogers, A. et al. [1998], "The meaning and management of neuroleptic medication: A study of patients with a diagnosis of schizophrenia," Social Science and Medicine 47(9):1313-1323). There are numerous reasons patients with psychotic disorders choose not to comply with a prescribed medication regimen. Between one-quarter and two-thirds of patients cite side effects as their primary reason for medication discontinuance (del Campo, E. J. et al. [1983], "Rehospitalized schizophrenics: what they report about illness, treatment and compliance," J. of Psychosocial Nurs. and Mental Health Serv. 21(6):29-33). Others cite lack of family support (Robinson et al., 2002). Other studies have directly linked the severity of psychopathology with non-compliance in both inpatient and outpatient settings (Fenton, W. S. et al. [1997], "Determinants of medication compliance in schizophrenia: empirical and clinical findings," Schizophrenia Bull. 23(4):637-651). The most powerful predictor of relapse and hospitalization among patients with schizophrenia is nonadherence with antipsychotic treatment regimens (Csernansky JG, Schuchart EK: Relapse and rehospitalization rates in patients with schizophrenia effects of second generation antipsychotics. CNS Drugs 2002, 16:473-484. Doering S, Muller E, Kopcke W, Pietzcker A, Gaebel W, Linden M, Muller P, Muller-Spahn F, Tegeler J, Schussler G: Predictors of relapse and rehospitalization in schizophrenia and schizoaffective disorder. Schizophr. Bull 1998, 24:87-98.). Other studies have reported an even greater incidences of non-compliance (up to 55%). In England, where adherence rates for neuroleptics converge at a 50% level, researchers concluded, "This rate of non-consumption of prescribed medications suggests that, for many individuals, non-compliance holds more benefits than compliance" (Rogers et al. [1998], supra, p. 1315).

With an economic and social burden of healthcare cost, for nonadherence in mental health estimated at $2.3 Billion annualy (Menzin et al., 2003), therapy that helps patients adhere to medication regimens for extensive periods of time, or alternatively, removes the patient from the decision making process, would improve clinical outcome substantially.

### SUMMARY OF THE INVENTION

In one embodiment, this invention provides a method of treating a Nervous System disorder in a subject in need thereof, the method comprising administering to the subject a first formulation of a drug in complex with a biodegradable polymer, wherein said formulation delivers said drug with pseudo zero-order kinetics *in-vivo;* and administering to the subject a second formulation of said drug in complex with a biodegradable polymer, wherein the second formulation delivers the drug at a faster rate, *in-vivo,* than the first formulation, whereby administering said first and second formulation results in therapeutic circulating levels of said drug, thereby being a method of treating a nervous system disorder.

In another embodiment, this invention provides a method comprising administering to the subject a first formulation of a drug in complex with a biodegradable polymer, wherein said formulation delivers said drug with pseudo zero-order kinetics *in-vivo*; and administering to the subject a second formulation of said drug in complex with a biodegradable polymer, wherein the second formulation delivers the drug at a faster rate, *in-vivo,* than the first formulation, whereby administering said first and second formulation results in therapeutic circulating levels of said drug indefinitely upon re implanting.

In another embodiment, the invention provides a kit for sustained delivery of a drug comprising a first formulation of said drug in complex with a biodegradable polymer, wherein said formulation delivers said drug with pseudo zero-order kinetics in-vivo, and a second formulation of said drug in complex with a biodegradable polymer wherein said second formulation has a rate of release which is faster than said first formulation, in-vivo.

In one embodiment, the present invention provides a composition for use in the treatment of psychotic disorders, comprising a poly(d,l-lactide/glycolide) (PLGA) copolymer at a concentration of from about 95-98% (w/w), and an antipsychotic agent (at a concentration of from about 2 to about 5% w/w), wherein the lactide:glycolide ratio of said poly(lactide/glycolide) copolymer is from about 100:0 to 50:50 and wherein said antipsychotic agent is Risperidone or 9-OH-Risperidone

### BRIEF DESCRIPTION OF THE DRAWINGS

FIG. 1 is a graph showing Haloperidol release for total of 443 days from PLGA disk implanted in monkeys.
FIG. 2A is a graph showing Haloperidol release for total of 379 days from single PLGA disk system implanted in Rabbits.
FIG. 2B is a graph showing Haloperidol release for total of 379 days from combination PLGA disk system implanted in Rabbits.
FIG. 3 is a graph showing *In-vitro* cumulative Risperidone release from implants containing expressed as total mass of Risperidone released.
FIG. 4A is a graph of cumulative release of 20, 40 or 60% (w/w) Risperidone dispersed in 85:15 PLGA released from 50 mg implant.
FIG. 4B is a graph of cumulative release of 20, 40 or 60% (w/w) Risperidone dispersed in 85:15 PLGA Release expressed as percentage of the amount of drug in each implant.
FIG. 5: is a graph showing cumulative *in-vitro* release from disk and rod-shaped implants. Differences in release profile were statistically insignifacnt.
FIG. 6: is a graph showing a model for continuous delivery from biodegradable implants projected to oscillate around a target serum level as long as implantations occur ecery 6 months.
FIG. 7A: is a graph showing expected serum release from a single polymer
FIG. 7B: is a graph showing expected serum release from reimplantation of a single polymer matrix every 6 months.
FIG. 7C: is a graph showing expected serum release pattern from reimplantation of a single polymer system every 6 months, along with a maintenance set with 3 month peak release.
FIG. 7D: is a graph showing the expected release from a starter polymer set, abridging the first 4 months of therapy.
FIG. 7E: is a graph showing the expected release from a combination therapy comprising starter polymer set, abridging the first 4 months of therapy with reimplantation of a single polymer system every 6 months, along with a maintenance set with 3 month peak release.
FIG. 8A is a graph showing 40% haloperidol release from Implants made from 75:25 PLGA:
FIG. 8b is a graph showing 40% haloperidol release from Implants made from 85:15 PLGA:
FIG. 8C: is a graph showing cumulative release from a hypothetical two-polymer system over a period of 154 days.
FIG. 9: is a graph showing locomotor activity (total distance in meters over twenty minutes) tested prior to implant removal and then again 48 hours following implant
FIG. 10 shows the Western blot of D2 receptor protein in striatal membranes from rats treated with haloperidol-PLA or PLA alone implants for 3 months.
FIG 11A is a graph of cumulative release of Risperidone dispersed in PLGA biodegradable matrix.
FIG. 11B is a graph of cumulative release of 9-OH-Risperidone dispersed in PLGA biodegradable matrix.
FIG. 12 is a graph of cumulative release of 20% (w/w) 9-OH-Risperidone dispersed in 85:15 PLA:PGA block copolymer biodegradable matrix.
FIG. 13 is a graph of cumulative release of 20% (w/w) Risperidone dispersed in 85:15 PLA:PGA block copolymer biodegradable matrix.
FIG. 14 A: Is a graph showing the effect of Implants in rats or mice for in vivo behavioral testing with prepulse inhibition of startle (PPI).
FIG 14B: Is a graph showing the effect of Implants in rats or mice for in vivo behavioral testing with prepulse inhibition of startle (PPI) following Amphetamine administration
FIG 14C: Is a graph showing the effect of Risperidone Implants in rats or mice for in vivo behavioral testing with prepulse inhibition of startle (PPI) pre and post Amphetamine administration.
FIG 14D: Is a graph showing the cumulative Risperidone release from 85:15 (PLA:PGA) PLGA implants as a function of time
FIG 14E: Is a graph showing the effect of Risperidone Implants in rats or mice on in vivo behavioral testing with prepulse inhibition of startle (PPI).
FIG 14F: Is a graph showing the effect of Risperidone Implants in rats or mice on in vivo behavioral testing with prepulse inhibition of startle (PPI) following vehicle injection
FIG. 14G: Is a graph showing the effect of Risperidone Implants in rats or mice on in vivo behavioral testing with prepulse inhibition of startle (PPI) following amphetamine administration.
FIG. 15A: Is a graph showing the cumulative quetiapine release from 85:15 (PLA:PGA) PLGA implants as a function of time.
FIG. 15B: Is a graph showing the effect of Quetiapine release from implant before and after injection of apomorphine, relative to control on PPI.
FIG. 15C: Is a graph showing the effect of Quetiapine injection before and after injection of apomorphine, relative to control and sham apomorhine injection on PPI.
FIG. 16A: Is a graph showing the the cumulative clozapine release from 85:15 (PLA:PGA) PLGA implants as a function of time.
FIG. 16B: Is a graph showing the effect of Clozapine Implants before and after placebo injection of apomorphine, relative to control and sham apomorhine injection on PPI.
FIG. 16C: Is a graph showing the effect of Clozapine Implants before and after injection of apomorphine, relative to control and sham apomorhine injection on PPI
FIG. 17A: Is a graph showing the cumulative haloperidol release from 85:15 (PLA:PGA) PLGA implants as a function of time.
FIG. 17B: Is a graph showing the effect of haloperidol release from PLGA implants on PPI.
FIG. 17C: Is a graph showing the effect of haloperidol Implants in rats or mice on in vivo behavioral testing with prepulse inhibition of startle (PPI)
FIG. 18A: Is a graph showing the effect of initial drug load on cumulative Risperidone release from 85:15 (PLA:PGA) PLGA implants *in-vitro*
FIG. 18B: Is a graph showing the effect of initial drug load on fractional Risperidone release from 85:15 (PLA:PGA) PLGA implants *in-vitro*

### DETAILED DESCRIPTION OF THE INVENTION

This invention provides in one embodiment, long-term sustained delivery formulations and methods of use thereof.

In one embodiment, this invention provides a method of treating a Nervous System disorder in a subject in need thereof, the method comprising administering to the subject a first formulation of a drug in complex with a biodegradable polymer, wherein said first formulation delivers said drug with pseudo zero-order kinetics *in-vivo;* and administering to said subject a second formulation of said drug in complex with a biodegradable polymer, wherein the second formulation delivers said drug substantially faster, *in-vivo,* than the first formulation, whereby administering said first and second formulation results in therapeutic circulating levels of said drug, thereby being a method of treating a nervous system disorder.

In another embodiment, this invention provides a method of treating a Nervous System disorder in a subject in need thereof, wherein the method uses a kit comprising the formulations as described further hereinbelow.

In one embodiment, the method may further comprise administrating at least one additional formulation comprising the drug in complex with a biodegradable polymer, wherein the rate of release of the drug from said one additional formula is faster than the first formulation and in one embodiment, is faster than the second formulation, or in another embodiment is slower than the second formulation, or in another embodiment is slower than the first formulation. In one embodiment, the one additional formulation has a peak drug release which is prior to the peak release of either the first formulation, or in another embodiment, the second formulation. In another formulation the one additional formulation has a peak drug release which coincides with the peak release of either the first formulation, or in another embodiment, the second formulation, or in another embodiment follows the peak release of said first formulation.

In one embodiment, the methods, kits and compositions of this invention, as further described hereinbelow comprise or make use of a first formulation, which is in complex with a biodegradable polymer that is metabolized, or in another embodiment produces non-toxic, or in another embodiment sub-pathologically toxic by-products.

The degradation products of PLGA, lactic acid and glycolic acid in one embodiment, are water soluble, non-toxic products of normal metabolism that are either excreted or further metabolized to carbon dioxide and water in the Krebs cycle.

In one embodiment, the terms "in complex" or "complexes" refer to the drug molecules being interdispersed within a biodegradable polymer's matrix. In one embodiment, the drug is located within interstitial spaces within the polymer matrix, which may be accomplished, in antoher embodiment, via fast solvent extraction, wherein, following extraction, a resulting increase in polymer concentration occurs, which causes a rapid increase in polymer viscosity, and in another embodiment, lowers diffusivity of the thus entrapped drug.

In one embodiment, the term "fast solvent extraction" refers to solvent removal at a rate in which the resulting increase in polymer concentration and subsequent increase in viscosity are such that allignment of the polymer chains to the point of recrystallization is kinetically unfeasible..

In one embodiment, any factor, which may affect the entrapment of the subject drug in the biodegradable polymer matrix, and thereby affect its initial loading, in one embodiment, or, in another embodiment, subsequent release, or in another embodiment, a combination thereof, may be utilized according to the methods of this invention, and in kits and compositions of this invention. In other embodiments, such factors may comprise *inter-alia,* the initial solvent concentration, its molecular size and polarity, the temperature and pressure under which the solvent is removed, molecular weight number (MWn) average of the biodegradable polymer matrix, its polydispersity index, the size and polarity of the drug, the monomer ratio and distribution along the copolymer's chain, or a combination thereof. In addition, D/L ratio within each monomer of a biodegradable polymer will affect release rates. In one embodiment, the term D/L ratio refers to the ratio of monomer molecules that affect the direction (D-right, L-left), in which a cross-polarized lense will be rotated when observing a single optically active monomer , like lactic acid. Since most mammals have D-specific enzymes, that ratio will affect the digestion rate of the biodegradable biopolymer, affecting its molecular weight and consequently its viscosity, thereby affecting release rate of any entrapped drug.

In one embodiment, the term "circulating level" refers to the concentration of the drug in blood serum as measured by separating the serum and determining drug concentration. In one embodiment, the term "therapeutic" means the produced desired biological, or in another embodiment, prophylactic response, as a consequence of a method, or administration of a kit or composition of this invention. In one embodiment, the desired response can be a reduction (complete or in another embodiment partial) of symptoms associated with a particular disease or disorder, such as, schizophrenia, depression, psychotic anxiety or combination thereof. In another embodiment, on the appearence of undesirable side effects (e.g. patients exhibit immunodeficiency from treatment), due to use of the method, kit or composition of this invention, this invention provides for the administration of appropriate palliative drugs during the treatment regimen.

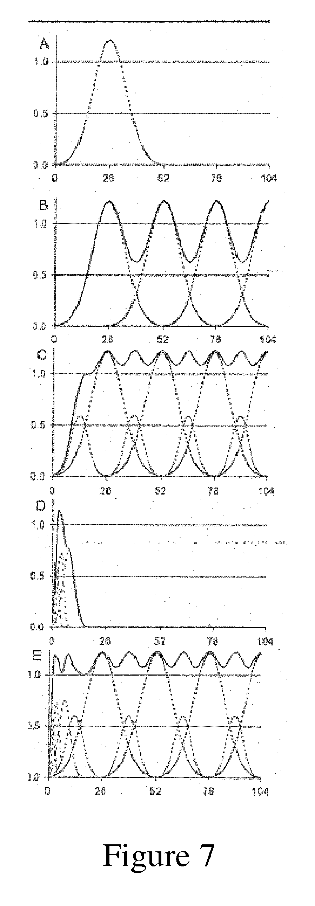

As an example as described in Example 12 hereinbelow, a nervous system disorder like schizophrenia can be treated with a drug, which in one embodiment, is Risperidone, by complexing the drug with a biodegradable polymer exhibiting the release profile shown in Figure 7A. In another embodiment, a formulation comprising a drug in complex with 5 different polymers may be used simultaneously wherein 3 complexes comprise a "starter set", referring in one embodiment to the formulations administered to bridge the lag of release from the sustained release implants (Figures 7D and 7E). According to this aspect of the invention, the starter set comprises 3 complexes the first of which has an immediate release profile, which is taken simultaneously with two other fast release complexes, each with a slightly slower release profile. In one embodiment, the starter set is taken concurrent with or just prior to the "maintenance set", which comprises at least one, or in another embodiment, 2, or in another embodiment, 3, or in another embodiment, 4 complexes, which provides for constant therapeutic circulating level of Risperidone. The maintenance set is provided at a point when complete release, referring in one embodiment to 100% of the drug is released from the starter set complexes. In another embodiment, the maintenance set may comprise a 5^{th} complex, which in one embodiment, is an implant comprising the drug and biodegradable matrix, which, has a higher absolute drug content, as compared to the other complexes. In one embodiment, the 5^{th} complex peak release coincides with a decline in drug release from the other complexes comprising the "maintenance set". According to this aspect of the invention, the release profiles from each complex are combined at an optimum to provide for a constant desired circulating Risperidone level (Figure 7E).

In one embodiment, the maintenance sets may be readministered to a subject indefinitely, to provide for prolonged therapy.

In another embodiment, the method of this invention includes repeated use, e.g. the use of the method according to the invention includes administration of one or more of the formulations as a repeated discrete treatment for a given period. In one embodiment the method of this invention includes cyclical use, e.g., one or more of the formulations of the invention may be administered at fixed time intervals. In one embodiment, the method may comprise evaluation of therapy midcourse, and formulations may be changed as a function of any sign of improvement of the subject, including lessening of symptoms, or in another embodiment, lessening of pathology, etc., as will be appreciated by one skilled in the art. In another embodiment, therapies may be altered following an indication of a lack of response to particular administered formulations.

In another embodiment, formulation of drug and polymer implants are designed without surfactants or emulsifiers, instead using solvent casting from acetone followed by compression molding. In one embodiment, acetone is chosen as the solvent for asting, or in another embodiment, other FDA Class III solvents (low toxicity with minimal need for removal of residual solvent) are used, in which, in one embodiment, the drug and biodegradable polymers are soluble at greater than 100 mg/ml. In another embodiment, implants thus produced are tolerable, and bioactive, and can be demonstrated as such, for example in rodents where a demonstration of tolerability and bioactivity following implantation in mouse and rat can be evaluated over a course of time, for example for 3 weeks to 3 months post-implantation.

In one embodiment solvent casting is accomplished with solvents chosen from **table I** (FDA Class III solvents (FDA Guidance document Q3C)):

**Table I FDA Class III solvents (FDA Guidance document Q3C**

| | | | |
|---|---|---|---|
| Acetic acid | Cumene | Heptane | 2-Methyl-1-propanol |
| Acetone | Dimethyl sulfoxide | Isobutyl acetate | Pentane |
| Anisole | Ethanol | Isopropyl acetate | 1-Pentanol |
| 1-Butanol | Ethyl acetate | Methyl acetate | 1-Propanol |
| 2-Butanol | Ethyl ether | 3-Methyl-1-butanol | 2-Propanol |
| Butyl acetate | Ethyl formate | Methylethyl ketone | Propyl acetate |
| tert-Butylmethyl ether | Formic acid | Methylisobutyl ketone | |

In another embodiment, the methods, kit and compositions of this invention provide for drug release profiles, which are a function of the interaction of each drug with the respective polymer. In another embodiment, other factors which may affect the patterns of release from the polymeric systems include drug diffusion rate, drug/polymer affinity, pH, source/sink concentrations, molecular weight average (MW_{w}), polymer number average (MWₙ), their ratio (Polydispersity index [PDI]) and the capability of physiological fluids to plasticize the biopolymer..

In one embodiment, the methods, kit and compositions of this invention provide that the first formulation and second formulation are administered within 1-48 hours of each other. In another embodiment, administration of the first formulation is done simultaneously with administration of the second formulation. In one embodiment, administration of the first formulation is done before administration of the second formulation. In another embodiment, administration of the first formulation is done after administration of the second formulation.

In one embodiment, the methods, kit and compositions of this invention make use of a biodegradable polymer, which is in one embodiment Poly(d,l-lactide-glycolide) copolymer [PLGA]. In one embodiment, the biodegradable PLGA polymers may be as described by ( Kitchell JP, Wise DL (1985) Poly(lactic/glycolic acid) biodegradable drug-polymer matrix systems. Methods Enzymol 112:436-448).

In one embodiment, the biodegradable polymer is a polylactide, a polyglycolide, a polycaprolactone, a copolymer thereof, a terpolymer thereof, or any combination thereof. In one embodiment the copolymer thereof is atactic, or in another embodiment syndiotactic. In one embodiment, the biodegradable thermoplastic polyester is a polylactide, a polyglycolide, a copolymer thereof, a terpolymer thereof, or a combination thereof.

In one embodiment the biodegradable thermoplastic polyester is 50/50 poly (DL-lactide-co-glycolide). In another embodiment, the biodegradable thermoplastic polyester is 75/25 poly (DL-lactide-co-glycolide). In another embodiment, the biodegradable thermoplastic polyester is 85/15 poly (DL-lactide-co-glycolide). In another embodiment, the biodegradable thermoplastic polyester is 60/40 poly (DL-lactide-co-glycolide). In another embodiment, the biodegradable thermoplastic polyester is 90/10 poly (DL-lactide-co-glycolide). In another embodiment, the biodegradable thermoplastic polyester comprises any combination of poly (DL-lactide-co-glycolide), which produces a desired release profile when in complex with a drug. In one embodiment, the biodegradable thermoplastic polyester can be present in any suitable amount, provided the biodegradable thermoplastic polyester is at least substantially insoluble in aqueous medium or body fluid.

In another embodiment the biodegradable thermoplastic polyester is preferably present in about 50 wt. % to about 98 wt. % of the flowable composition, or in one embodiment is present in about 50 wt. % to about 60 wt. % of the flowable composition, or in another embodiment in about 60 wt. % to about 75 wt. % of the flowable composition, or in another embodiment in about 75 wt. % to about 90 wt. % of the flowable composition, or in another embodiment, in about 90 wt. % to about 95 wt. % of the flowable composition, or in another embodiment, in about 95 wt. % to about 98 wt. % of the flowable composition.

In one embodiment, the biodegradable thermoplastic polymer has an average molecular weight of about 10,000 to about 200,000, or in another embodiment from about 15,000 to about 25,000, or in another embodiment from about 25,000 to about 45,000, or in another embodiment from about 45,000 to about 75,000, or in another embodiment from about 75,000 to about 100,000, or in another embodiment from about 100,000 to about 150,000, or in another embodiment from about 150,000 to about 200,000.

In one embodiment, the lactic monomer comprising the PLGA is at a concentration of between 50-100%, or in another embodiment between 50-60%, or in another embodiment between 60-70%, or in another embodiment between 70-80%, or in another embodiment, between 80-90%, or in another embodiment, between 90-100% of the PLGA polymer, or in another embodiment comprises 100% PLA.

In one embodiment, the term "about" refers to a deviation from the range of 1-20%, or in another embodiment, of 1-10%, or in another embodiment of 1-5%, or in another embodiment, of 5-10%, or in another embodiment, of 10-20%.

In one embodiment, the drug-polymer complex in a given formulation is such that the drug content is between 2 and 75% (w/w), or in another embodiment between 2 and 5% (w/w), or in another embodiment between 5 and 10% (w/w), or in another embodiment between 10 and 25% (w/w), or in another embodiment between 25 and 35% (w/w), or in another embodiment between 35 and 50% (w/w), or in another embodiment between 50 and 75% (w/w).

In one embodiment, the complexes may be formed via covalent attachment of watersoluble polymers such as polyethylene glycol, copolymers of polyethylene glycol and polypropylene glycol, carboxymethyl cellulose, dextran, polyvinyl alcohol, polyvinylpyrrolidone or polyproline. In one embodiment modifications may increase the compound's solubility in aqueous solution, eliminate aggregation, enhance the physical and chemical stability of the compound, reduce the immunogenicity or reactivity of the compound or combination thereof.

In another embodiment, the methods, kit and compositions of the present invention provide for the treatment of any disease or disorder wherein delivery of at least one drug over a sustained period of time is desired.

It is to be understood that any condition, disease or disorder, whereby a treatment regimen with a particular compound or combination of compounds, which comprises providing an initial formulation, which achieves therapeutic circulating levels, of the particular compound or combination of compounds, which is concurrent with or followed by treatment with a second formulation, which achieves therapeutic circulating levels, wherein the circulating levels attained by the second formulation, coincide with decline of such levels by the first formulation, and the circulating levels are achieved over a prolonged course of time, is to be considered as part of this invention.
The first formulation of the methods, kit and compositions of the present invention, deliver the drug with pseudo-zero order kinetics which in one embodiment refers to a "steady state" (zero order) release profile, or almost steady state, or in one embodiment with a variation of ±5-10% from steady state, which delivers a therapeutically effective amount of drug over a given time period. In another embodiment, oscillation of ±5%, or in another embodiment ±10%, or in another embodiment ±20%, or in another embodiment ±30% is observed around a target serum level. In one embodiment, the time period is from 3-10 months post administration. In one embodiment, the time period is from 14 days-6 months post administration.

In one embodiment, the release profile from the formulation used in the methods, kit and compositions of the present invention may parallel that shown in Figure 7A, or in another embodiment Figure 7B, or in another embodiment Figure 7C, or in another embodiment, Figure 7C, or in another embodiment Figure 7D or in another embodiment Figure 7E, or in another embodiment, approximate such profiles.

In one embodiment, the formulation may be administered as a controlled release patch. In another embodiment, the subject therapeutic drug is delivered by way of a transdermal patch. A patch is in one embodiment, a flat hollow device with a permeable membrane on one side, and, also may comprise an adhesive to maintain the patch in place on the patient's skin, with the membrane in contact with the skin so that the medication can permeate out of the patch reservoir and into and through the skin. The outer side the patch is formed, in one embodiment of an impermeable layer of material, and the membrane side and the outer side are joined around the perimeter of the patch, forming a reservoir for the medication and carrier between the two layers. Patch technology enables the active ingredient to be in constant contact with the epidermis. Over substantial periods of time, drug molecules, held in such a state, will diffuse into the bloodstream, due, in one embodiment to a concentration gradient. in another embodiment, transdermal drug delivery can be accomplished using patch technology. These conventional drug delivery systems comprise a patch with an active ingredient such as a drug incorporated therein, the patch also including an adhesive for attachment to the skin so as to place the active ingredient in close proximity to the skin. Exemplary patch technologies are available from Ciba-Geigy Corporation and Alza Corporation. Such transdermal delivery devices can be readily adapted for use with the subject amphetamine compounds. In one embodiment, a maintenance set of the drug is administered once every 3 months in a patch form.

In one embodiment, the formulations are in a form of an implant. In another embodiment, release from and in another embodiment diffusion of the drug from the implant vary substantially for different drugs. In one embodiment design specifications for one compound may not be directly translated to another.

In one embodiment an antipsychotic, or in another embodiment 9-OH-Risperidone are used as part of the methods, kit and compositions of the present invention to treat a Nervous System disorder, which in one embodiment is Bipolar disorder.

In one embodiment, the methods, kits and compositions of this invention may be used in order to deliver thyrotropin-releasing hormone, or L-dopa, in order to treat Parkinson's Disease. In another embodiment, the methods, kits and compositions of this invention may be used in order to deliver naltrexone , in order to treat narcotic addiction.

It is to be understood that the formulations used in the methods, kit and compositions of the present invention, may comprise combination of drugs, which in one embodiment may be a part of the treatment regimen of the disorder.

In one embodiment, the present invention provides a method for treating nervous system disorders, wherein the drug is at a concentration of between about 2 to about 50 percent by weight of said first or second formulation. In another embodiment, the drug is at a concentration of between about 2 to about 5 percent by weight of said first or second formulation. In one embodiment, the drug is at a concentration of between about 5 to about 10 percent by weight of said first or second formulation. In another embodiment, the drug is at a concentration of between about 10 to about 15 percent by weight of said first or second formulation. In one embodiment, the drug is at a concentration of between about 15 to about 20 percent by weight of said first or second formulation. In another embodiment, the drug is at a concentration of between about 20 to about 30 percent by weight of said first or second formulation. In one embodiment, the drug is at a concentration of between about 30 to about 40 percent by weight of said first or second formulation. In another embodiment, the drug is at a concentration of between about 40 to about 50 percent by weight of said first or second formulation.

In one embodiment, the present invention provides a method for treating nervous system disorders, wherein the drug used is Risperidone, 9-OH-Risperidone, haloperidol, olanzapine, clozapine, aripiprazole, quetiapine, ziprasidone or a combination thereof

In one embodiment, the first and second formulation have at least one drug in-common.

In another embodiment the first formulation comprises Risperidone and 9-OH-Risperidone and the second formulation comprises 9-OH-Risperidone, or in another embodiment, the first formulation comprises Risperidone and haloperidol, and the second formulation comprises haloperidol, or in another embodiment the first formulation comprises quetiapine and 9-OH-Risperidone and the second formulation comprises 9-OH-Risperidone.

In one embodiment, the first formulation contain drugs that are therapeutic in chronic diseases. In another embodiment, the second formulation comprises drug that are therapeutic for an acute phase of the chronic disease. For example, in one embodiment, relapsing and remitting diseases such as Multiple Sclerosis, may thus be treated, where in one embodiment, each formulation will comprise Copaxon, while the first formulation may additionally comprise -interferon, or in another embodiment, an immunomodulating compound.

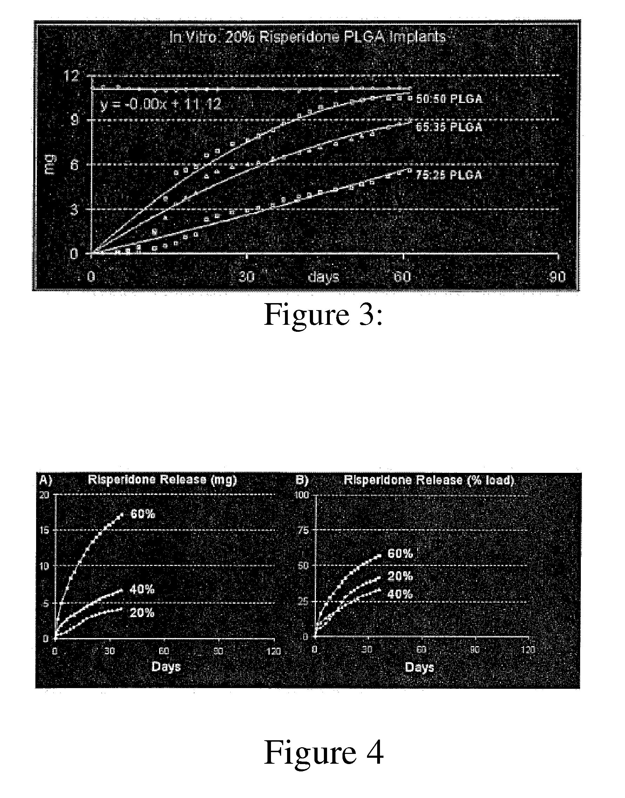

In another embodiment, the second formulation has a rate of release which is faster than the first formulation. In one embodiment the term "faster rate of release refers to the increase in relative concentration of circulating serum levels of the subject drug, as a function of time. In one embodiment, the % drug released as a function of time is graphically depicted in Figure 4B.

In another embodiment, the second formulation may be administered as a depot injection. In another embodiment the second formulation is administered as microspheres containing the drug entrapped within the biodegradable polymer matrix.

In one embodiment, the first formulation of the methods, kit and composaitions of the prsent invention is such that the rate of release of drugs is substantially slow, in order to provide for prolonged sustained release. Substantially slow, refers in one embodiment to an order of magnitude, relative to an average under normal formulation.

In one embodiment, the present invention provides a method for treating nervous system disorders, wherein the first formulation is an implant, and is administered subcutaneously, and said second formulation comprises oral drug form, a parenteral form, or an intravenous form.

In one embodiment, the phrases "administered parenterally" as used herein means mode of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, intratumoral, subcapsular, subarachnoid, intraspinal and intrasternal injection and infusion.

In another embodiment, the present invention provides for the cyclic administration of the first and second formulations to said subject. It is to be understood that order of administartion of first, second or in another embodiment, any additional administration of formulas containing the drug is within the scope of the present invention and may be varied to provide a designed circulating level of the drug, as will be appreciated by one skilled in the art.

In one embodiment, the first and second formulations are administered to said subject when the circulating levels of the drug serum levels are below the therapeutic threshold level for that drug. In another embodiment, the first and second formulations are administered to said subject when the circulating levels of said drug serum levels are below 1 ng/mL.

In another embodiment, the present invention provides a method for treating nervous system disorders, wherein the first and second formulations are administered to said subject as described hereinabove from about 160-200 days, following a first administration of the formulations. In one embodiment the first and second formulations are administered to said subject from about 160-180 days, following a first administration of the formulations. In another embodiment, the first and second formulations are administered to said subject from about 180-200 days, following a first administration of the formulations.

In another embodiment, the present invention provides a method for treating nervous system disorders, wherein the method addresses the need for more sustained treatment adherence, or in one embodiment a methods of long-term medication delivery using implantable systems for the treatment of schizophrenia. Implantable systems have in another embodiment, the capability to optimize a medication's therapeutic properties, which in one embodiment renders treatments that are more safe, efficacious or reliable. In another embodiment, the present invention provides a method for treating nervous system disorders, wherein less medication may be generally required and side effects can be minimized. In one embodiment implantable systems can be removed by a physician in case of adverse side effects, offering a degree of reversibility. In another embodiment, the present invention provides a method for treating nervous system disorders, wherein mandatory removal at the end of the delivery interval is eliminated with biodegradable systems, cutting in half the inherent invasiveness to the patient.

In one embodiment, the present invention provides a method for treating nervany diseases or disorders where a prolonged therapy period is needed. In one embodiment the drugs are used to treat: HIV and other viral diseases, mycobacterial infection, cancer, multiple sclerosis, diabetes, kidney disease, or any other wherein the methods, kits or compositions of this invention prove useful.

In another embodiment, the present invention provides a method for treating nervous system disorders, wherein said disorder is AIDS -related dementia, schizophrenia, bipolar disorder, borderline personality disorder (BPD), Alzheimer's disease (AD), psychotic depression or other mental disorders causing confusion, disorganization or psychosis.

In one emboidment, the drug used according to the methods, kits or compositions of this invention, may be any drug which is applicable for such treatment regimens, as described herein. In one embodiment, the drug is a peptide, protein, nucleic acid, or compound.

In one embodiment, the term "drug" refers to a molecule that alleviates a symptom, disease or disorder when administered to a subject afflicted thereof. In one embodiment, a drug is a synthetic molecule, or in another embodiment, a drug is a naturally occurring compound isolated from a source found in nature.

In one embodiment, drugs may comprise antihypertensives, antidepressants, antianxiety agents, anticlotting agents, anticonvulsants, blood glucose-lowering agents, decongestants, antihistamines, antitussives, anti-inflammatories, antipsychotic agents, cognitive enhancers, cholesterol-reducing agents, antiobesity agents, autoimmune disorder agents, anti-impotence agents, antibacterial and antifungal agents, hypnotic agents, anti-Parkinsonism in agents, antibiotics, antiviral agents, anti-neoplastics, barbituates, sedatives, nutritional agents, beta blockers, emetics, anti-emetics, diuretics, anticoagulants, cardiotonics, androgens, corticoids, anabolic agents, growth hormone secretagogues, anti-infective agents, coronary vasodilators, carbonic anhydrase inhibitors, antiprotozoals, gastrointestinal agents, serotonin antagonists, anesthetics, hypoglycemic agents, dopaminergic agents, anti-Alzheimer's Disease agents, anti-ulcer agents, platelet inhibitors and glycogen phosphorylase inhibitors.

According to this aspect of the invention, and in one embodiment, examples of the drugs used according to this invention include, *inter-alia,* antihypertensives including prazosin, nifedipine, trimazosin, amlodipine, and doxazosin mesylate; the antianxiety agent hydroxyzine; a blood glucose lowering agent such as glipizide; an anti-impotence agent such as sildenafil citrate; anti-neoplastics such as chlorambucil, lomustine or echinomycin; anti-inflammatory agents such as betamethasone, prednisolone, piroxicam, aspirin, flurbiprofen and (+)-N-{4-[3-(4-fluorophenoxy)phenoxy]-2-cyclopenten-1-yl}-N-hyroxyurea; antivirals such as acyclovir, nelfinavir, or virazole; vitamins/nutritional agents such as retinol and vitamin E; emetics such as apomorphine; diuretics such as chlorthalidone and spironolactone; an anticoagulant such as dicumarol; cardiotonics such as digoxin and digitoxin; androgens such as 17-methyltestosterone and testosterone; a mineral corticoid such as desoxycorticosterone; a steroidal hypnotic/anesthetic such as alfaxalone; an anabolic agent such as fluoxymesterone or methanstenolone; antidepression agents such as fluoxetine, pyroxidine, venlafaxine, sertraline, paroxetine, sulpiride, [3,6-dimethyl-2-(2,4,6-trimethyl-phenoxy)-pyridin-4-yl]-(lethylpropyl)-amine or 3,5-dimethyl-4-(3'-pentoxy)-2-(2',4',6'-trimethylphenoxy)pyridine; an antibiotic such as ampicillin and penicillin G; an anti-infective such as benzalkonium chloride or chlorhexidine; a coronary vasodilator such as nitroglycerin or mioflazine; a hypnotic such as etomidate; a carbonic anhydrase inhibitor such as acetazolamide or chlorzolamide; an antifungal such as econazole, terconazole, fluconazole, voriconazole or griseofulvin; an antiprotozoal such as metronidazole; an imidazole-type anti-neoplastic such as tubulazole; an anthelmintic agent such as thiabendazole or oxfendazole; an antihistamine such as astemizole, levocabastine, cetirizine, or cinnarizine; a decongestant such as pseudoephedrine; antipsychotics such as fluspirilene, penfluridole, risperidone or ziprasidone; a gastrointestinal agent such as loperamide or cisapride; a serotonin antagonist such as ketanserin or mianserin; an anesthetic such as lidocaine; a hypoglycemic agent such as acetohexamide; an anti-emetic such as dimenhydrinate; an antibacterial such as cotrimoxazole; a dopaminergic agent such as L-DOPA; anti-Alzheimer agents such as THA or donepezil; an anti-ulcer agent/H2 antagonist such as famotidine; a sedative/hypnotic such as chlordiazepoxide or triazolam; a vasodilator such as alprostadil; a platelet inhibitor such as prostacyclin; an ACE inhibitor/antihypertensive such as enalaprilic acid or lisinopril; a tetracycline antibiotic such as oxytetracycline or minocycline; a macrolide antibiotic such as azithromycin, clarithromycin, erythromycin or spiramycin; and glycogen phosphorylase inhibitors such as [R-(R*S*)]-5-chloro-N-[2-hydroxy-3{methoxymethylamino]-3-oxo-I-(phenylmethyl)-propyl]-IH-indole-2-carboxamide or 5-chloro-1 -Hindole-2-carboxylic acid [(IS)-benzyl(2R)-hydroxy-3-((3R,4S)dihydroxy-pyrrolidin-1-yl-)-oxypropyl]amide.

Further examples of drugs for use according to this invention are the glucose-lowering drug chlorpropamide, the anti-fungal fluconazole, the anti-hypercholesterolemic atorvastatin calcium, the antipsychotic thiothixene hydrochloride, the anxiolytics hydroxyzine hydrochloride or doxepin hydrochloride, the anti-hypertensive amlodipine besylate, the antiinflammatories piroxicam and celicoxib and valdicoxib, and the antibiotics carbenicillin indanyl sodium, bacampicillin hydrochloride, troleandomycin, and doxycycline hyclate.

In another embodiment a drug of this invention may comprise other antineoplastic agents such as platinum compounds (e.g., spiroplatin, cisplatin, and carboplatin), methotrexate, fluorouracil, adriamycin, mitomycin, ansamitocin, bleomycin, cytosine arabinoside, arabinosyl adenine, mercaptopolylysine, vincristine, busulfan, chlorambucil, melphalan (e.g., PAM, L-PAM or phenylalanine mustard), mercaptopurine, mitotane, procarbazine hydrochloride dactinomycin (actinomycin D), daunorubicin hydrochloride, doxorubicin hydrochloride, paclitaxel and other taxenes, rapamycin, manumycin A, TNP-470, plicamycin (mithramycin), aminoglutethimide,estramustine phosphate sodium, flutamide, leuprolide acetate, megestrol acetate, tamoxifen citrate, testolactone, trilostane, amsacrine (m-AMSA), asparaginase (L-asparaginase) Erwina asparaginase, interferon .alpha.-2a, interferon .alpha.-2b, teniposide (VM-26), vinblastine sulfate (VLB), vincristine sulfate, bleomycin sulfate, hydroxyurea ,procarbazine, and dacarbazine; mitotic inhibitors such as etoposide, colchicine, and the vinca alkaloids, radiopharmaceuticals such as radioactive iodine and phosphorus products; hormones such as progestins, estrogens and antiestrogens; anti-helmintics, antimalarials, and antituberculosis drugs; biologicals such as immune serums, antitoxins and antivenoms; rabies prophylaxis products; bacterial vaccines; viral vaccines; respiratory products such as xanthine derivatives theophylline and aminophylline; thyroid agents such as iodine products and anti-thyroid agents; cardiovascular products including chelating agents and mercurial diuretics and cardiac glycosides; glucagon; blood products such as parenteral iron, hemin, hematoporphyrins and their derivatives; biological response modifiers such as muramyldipeptide, muramyltripeptide, microbial cell wall components, lymphokines (e.g., bacterial endotoxin such as lipopolysaccharide, macrophage activation factor), subunits of bacteria (such as Mycobacteria, Corynebacteria), the synthetic dipeptide N-acetyl-muramyl-L-alanyl-D-isoglutamine; anti-fungal agents such as ketoconazole, nystatin, griseofulvin, flucytosine (5-fc), miconazole, amphotericin B, ricin, cyclosporins, and ß-lactam antibiotics (e.g., sulfazecin); hormones such as growth hormone, melanocyte stimulating hormone, estradiol, beclomethasone dipropionate, betamethasone, betamethasone acetate and betamethasone sodium phosphate, vetamethasone disodium phosphate, vetamethasone sodium phosphate, cortisone acetate, dexamethasone, dexamethasone acetate, dexamethasone sodium phosphate, flunisolide, hydrocortisone, hydrocortisone acetate, hydrocortisone cypionate, hydrocortisone sodium phosphate, hydrocortisone sodium succinate, methylprednisolone, methylprednisolone acetate, methylprednisolone sodium succinate, paramethasone acetate, prednisolone, prednisolone acetate, prednisolone sodium phosphate, prednisolone tebutate, prednisone, triamcinolone, triamcinolone acetonide, triamcinolone diacetate, triamcinolone hexacetonide, fludrocortisone acetate, oxytocin, vassopressin, and their derivatives; vitamins such as cyanocobalamin neinoic acid, retinoids and derivatives such as retinol palmitate, and .alpha.-tocopherol; peptides, such as manganese super oxide dismutase; enzymes such as alkaline phosphatase; anti-allergic agents such as amelexanox; anti-coagulation agents such as phenprocoumon and heparin; circulatory drugs such as propranolol; metabolic potentiators such as glutathione; antituberculars such as para-aminosalicylic acid, isoniazid, capreomycin sulfate cycloserine, ethambutol hydrochloride ethionamide, pyrazinamide, rifampin, and streptomycin sulfate; antivirals such as amantadine azidothymidine (AZT, DDI, Foscarnet, or Zidovudine), ribavirin and vidarabine monohydrate (adenine arabinoside, ara-A); antianginals such as diltiazem, nifedipine, verapamil, erythritol tetranitrate, isosorbide dinitrate, nitroglycerin (glyceryl trinitrate) and pentaerythritol tetranitrate; anticoagulants such as phenprocoumon, heparin; antibiotics such as dapsone, chloramphenicol, neomycin, cefaclor, cefadroxil, cephalexin, cephradine erythromycin, clindamycin, lincomycin, amoxicillin, ampicillin, bacampicillin, carbenicillin, dicloxacillin, cyclacillin, picloxacillin, hetacillin, methicillin, nafcillin, oxacillin, penicillin including penicillin G and penicillin V, ticarcillin rifampin and tetracycline; antiinflammatories such as diflunisal, ibuprofen, indomethacin, meclofenamate, mefenamic acid, naproxen, oxyphenbutazone, phenylbutazone, piroxicam, sulindac, tolmetin, aspirin and salicylates; antiprotozoans such as chloroquine,hydroxychloroquine, metronidazole, quinine and meglumine antimonate; antirheumatics such as penicillamine; narcotics such as paregoric;opiates such as codeine, heroin, methadone, morphine and opium; cardiac glycosides such as deslanoside, digitoxin, digoxin, digitalin and digitalis; neuromuscular blockers such as atracurium mesylate, gallamine triethiodide, hexafluorenium bromide, metocurine iodide, pancuronium bromide, succinylcholine chloride (suxamethonium chloride), tubocurarine chloride and vecuronium bromide; sedatives (hypnotics) such as amobarbital, amobarbital sodium, aprobarbital, butabarbital sodium, chloral hydrate, ethchlorvynol, ethinamate, flurazepam hydrochloride, glutethimide, methotrimeprazine hydrochloride, methyprylon, midazolam hydrochloride, paraldehyde, pentobarbital, pentobarbital sodium, phenobarbital sodium, secobarbital sodium, talbutal, temazepam and triazolam; local anesthetics such as bupivacaine hydrochloride, chloroprocaine hydrochloride, etidocaine hydrochloride, lidocaine hydrochloride, mepivacaine hydrochloride, procaine hydrochloride and tetracaine hydrochloride; general anesthetics such as droperidol, etomidate, fentanyl citrate with droperidol, ketamine hydrochloride, methohexital sodium and thiopental sodium; and radioactive particles or ions such as strontium, iodide rhenium and yttrium.

In one embodiment, the methods, kits and compositions of this invention provide for combined use of drugs, as described herein, wherein such combinations may differ within the respective formulations, so long as a single drug is in common amongst formulations administered to a subject, at one point in time.

In one embodiment, the methods of this invention may be affected via the use of a kit comprising the forumulations as described.

In one embodiment, the invention provides a kit for sustained delivery of at least one drug comprising a first formulation of said drug in complex with a biodegradable polymer, wherein said formulation delivers said drug with pseudo zero-order kinetics in-vivo, and a second formulation of said drug in complex with a biodegradable polymer wherein said second formulation has a rate of release which is faster than said first formulation, in-vivo.

In another embodiment, the invention provides a kit for sustained delivery of a drug, wherein the combination of the first and second formulation of the kit gives sustained delivery, once administered over a period of about 1 week to about 14 months. In one embodiment, the combination of the first and second formulation of the kit gives sustained delivery, once administered over a period of about 1 week to about 1 month. In another embodiment, the combination of the first and second formulation of the kit gives sustained delivery, once administered over a period of about 1 to about 3 months. In one embodiment, the combination of the first and second formulation of the kit gives sustained delivery, once administered over a period of about 3 to about 6 months. In another embodiment, the combination of the first and second formulation of the kit gives sustained delivery, once administered over a period of about 6 to about 9 months. In another embodiment, the combination of the first and second formulation of the kit gives sustained delivery, once administered over a period of about 9 to about 12 months. In another embodiment, the combination of the first and second formulation of the kit gives sustained delivery, once administered over a period of about 12 to about 14 months.

In one embodiment, the kit and compositions as described herein are used according to the methods of this invention, and in another embodiment, such use may include combination with other kits or in another embodiment, compositions as a part of systemic therapy, and in another embodiment, may comprise use of only part of a given kit, or particular formulations therein contained.

It is to be understood that the kit of the present invention may be used with any of the methods described hereinabove, and in another embodiment, may make use of any of the compositions or formulations described herein.

In one embodiment, the present invention provides a composition for use in the treatment of psychotic disorders, comprising a poly(d,l-lactide/glycolide) (PLGA) copolymer at a concentration of from about 95-98% (w/w), and an antipsychotic agent, at a concentration of from about 2 to about 5% (w/w), wherein the lactide:glycolide ratio of said poly(lactide/glycolide) copolymer is from about 100:0 to 50:50 and wherein said antipsychotic agent is Risperidone or 9-OH-Risperidone.

The formulations of the present invention may be given in one embodiment orally, parenterally, topically, or subcutaneously. In another embodiment the formulations are given by forms suitable for each administration route. For example in one embodiment, they are administered in tablets or capsule form, or in another embodiment by injection, infusion, inhalation, eye lotion, ointment, rectal suppository, or controlled release patch.

In one embodiment, the therapeutic circulating levels of said drug range from 0.1 - 10 ng/mL. In another embodiment, the therapeutic circulating levels of said drug range from 0.1 - 0.5 ng/mL. In one embodiment, the therapeutic circulating levels of said drug range from 0.5 - 1.0 ng/mL. In another embodiment, the therapeutic circulating levels of said drug range from 1.0 - 1.5 ng/mL. In one embodiment, the therapeutic circulating levels of said drug range from 1.5 - 2.5 ng/mL. In another embodiment, the therapeutic circulating levels of said drug range from 2.5- 5 ng/mL. In one embodiment, the therapeutic circulating levels of said drug range from 5-10 ng/mL.

In one embodiment, this invention provides, wherein administering the second formulation results in said circulating levels within a period of about 1-31 days. In another embodiment, administering the second formulation results in said circulating levels within a period of about 1-7 days. In one embodiment, administering the second formulation results in said circulating levels within a period of about 7-14 days. In another embodiment, administering the second formulation results in said circulating levels within a period of about 14-21 days. In one embodiment, administering the second formulation results in said circulating levels within a period of about 22-31 days.

In one embodiment, this invention, wherein administering the first formulation results in acheiving circulating levels within a period of about 21-180 days. In another embodiment, administering the first formulation results in acheiving circulating levels within a period of about 21-31 days. In one embodiment, administering the first formulation results in acheiving circulating levels within a period of about 31-60 days. In another embodiment, administering the first formulation results in acheiving circulating levels within a period of about 60-90 days. In one embodiment, administering the first formulation results in acheiving circulating levels within a period of about 90-120 days. In another embodiment, administering the first formulation results in acheiving circulating levels within a period of about 120-150 days. In another embodiment, administering the first formulation results in acheiving circulating levels within a period of about 150-180 days.

In one embodiment, this invention provides for circulating levels of at least one desired drug, which is sustained for about from 1-420 days. In one embodiment, this invention provides for circulating levels of at least one desired drug, which is sustained for about from 14-420 days. In one embodiment, this invention provides, wherein said circulating levels are sustained for about from 75-420 days. In another embodiment, said circulating levels are sustained for about from 75-180 days. In one embodiment, said circulating levels are sustained for about from 180-270 days. In another embodiment, said circulating levels are sustained for about from 270-365 days. In one embodiment, said circulating levels are sustained for about from 365-420 days.

In another embodiment, the present invention provides for the use of implants inserted subcutaneously. In one embodiment, procedures for implantation could be easily tolerated with local anesthetic before subcutaneous placement. In another embodiment, using rigid implants obviate the need for a tool to guide implants under the skin, reducing risk of intramuscular placement.

In another embodiment, the formulations may be such as to be suitable for implantation at any desired tissue site, in the form of a patch, or for injection, intravenously, intracavitarily, intranodally, or any other appropriate site, and may be in the form of a depot injection. In another embodiment, the route of administration and intervals between administration may be individually adjusted for a given subject. In one embodiment, the time interval may involve administration of the formulations once every six months. In another embodiment, treatment regimens according to the methods of this invention, and using the kits/compositions of this invention may be accompanied by other forms of treatment, which do not involve administration of a drug, such as subject interaction with psychiatrists and/or therapists.

In one embodiment, the present invention provides a method for treating a disease or disorder, wherein at least one formulation is in the form of an implant, which in one embodiment is is disk shaped, or, in another embodiment, rod shaped. In another embodiment, Polymers and drugs are mixed in a proportion of 60/40 by mass and solvent cast from acetone. The resulting film may be compression molded to disk-shaped implants of 20 mm diameter with average thickness of 1.22 ± 0.0 mm, mass of 493 ± 2 mg and density of 1.28 ± 0.0 g/cc, such as for example, the implants exemplified hereinbelow. In one embodiment, Rod-shaped implants are prepared as described in example 10 hereinbelow, having in another embodiment a diameter of about 1 to about 2 mm, a length of between about 10 and about 40 mm, or a combination thereof. It is to be understood that the geometry of the implant may be varied to any form which provides for a desired release profile, as will be appreciated by one skilled in the art, with dimensions which may vary in order to accommodate, in one embodiment, a desired drug load, or in another embodiment, to suit the environment wherein the material will be implanted, or any other consideration which will affect the methods of this invention, as will be appreciated by one skilled in the art.

In one embodiment, the invention provides a kit for sustained delivery of a drug comprising a first formulation of said drug in complex with a biodegradable polymer, wherein said formulation delivers said drug with pseudo zero-order kinetics in-vivo, and a second formulation of said drug in complex with a biodegradable polymer wherein said second formulation has a rate of release which is faster than said first formulation, in-vivo.

In another embodiment, the invention provides a kit for sustained delivery of a drug, wherein the combination of the first and second formulation of the kit gives sustained delivery, once administered over a period of about 1 week to about 14 months. In one embodiment, the combination of the first and second formulation of the kit gives sustained delivery, once administered over a period of about 1 week to about 1 month. In another embodiment, the combination of the first and second formulation of the kit gives sustained delivery, once administered over a period of about 1 to about 3 months. In one embodiment, the combination of the first and second formulation of the kit gives sustained delivery, once administered over a period of about 3 to about 6 months. In another embodiment, the combination of the first and second formulation of the kit gives sustained delivery, once administered over a period of about 6 to about 9 months. In another embodiment, the combination of the first and second formulation of the kit gives sustained delivery, once administered over a period of about 9 to about 12 months. In another embodiment, the combination of the first and second formulation of the kit gives sustained delivery, once administered over a period of about 12 to about 14 months.

In one embodiment, the kit and compositions of the formulations described hereinbelow are used in the methods described above in their entirety, partially or in combination with other kits or in another embodiment, composition as a part of systemic therapy.

In one embodiment, the invention provides a kit for sustained delivery of a drug, wherein the formulation is in a form of a rod-shaped implant as described in one embodiment hereinabove, said rod-shaped disk has a diameter of about 1 to about 2 mm, a length of between about 10 and about 40 mm, or a combination thereof.

In one embodiment, the present invention provides a flowable composition that is suitable for use as a controlled release implant of Risperidone or 9-OH-Risperidone. In another embodiment, the flowable composition includes a biodegradable thermoplastic polyester that is at least initially substantially insoluble in an aqueous medium or body fluid. In one embodiment, the flowable composition is formulated as an injectable subcutaneous delivery system. The injectable composition preferably has in another embodiment a volume of about 0.20 mL to about 0.40 mL or about 0.30 mL to about 0.50 mL. The injectable composition is preferably formulated for administration about once per month, or in another embodiment about once per three months, or in another embodiment about once per four months to about once per six months. Preferably, the flowable composition is a liquid or a gel composition, suitable for injection into a patient.

In one embodiment, the present invention provides a composition for use in the treatment of psychotic disorders, in the form of microparticles, which in another embodiment, are fabricated as described in example 11 hereinbelow. In another embodiment, the microparticles of the present invention have a mean diameter (D_{3,2}) of about 150 µm ± 50 µm, or in another embodiment a mean diameter (D_{3,2}) of about 0.75 ± 25 nm. In another embodiment, the formulations of this invention, for use in the methods or incorporation in the kits or compositions of this invention may comprise nanoparticles, which comprise the drug in complex with a biodegradable matrix, as herein described.

The comp[ositions of the present invention may be administered in one embodiment to humans and other animals for therapy by any suitable route of administration, including orally, nasally, as by, for example, a spray, rectally, intravaginally, parenterally, intracisternally and topically, as by powders, ointments or drops, including buccally and sublingually.

In one embodiment, the composition can be delivered in a controlled release system. For example, the agent may be administered using intravenous infusion, an implantable osmotic pump, a transdermal patch, liposomes, or other modes of administration. In one embodiment, a pump may be used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1989). In another embodiment, polymeric materials can be used. In another embodiment, a controlled release system can be placed in proximity to the therapeutic target, i.e., the brain, thus requiring only a fraction of the systemic dose (see, e.g., Goodson, in Medical Applications of Controlled Release, supra, vol. 2, pp. 115-138 (1984). Other controlled release systems are discussed in the review by Langer (Science 249:1527-1533 (1990).

In another embodiment, the composition is in a form suitable for oral, intravenous, intraaorterial, intramuscular, subcutaneous, parenteral, transmucosal, transdermal, or topical administration. In one embodiment the composition is a controlled release composition. In another embodiment, the composition is an immediate release composition. In one embodiment, the composition is a liquid dosage form. In another embodiment, the composition is a solid dosage form.

In one embodiment, the compositions of this invention may be in the form of a pellet, a tablet, a capsule, a solution, a suspension, a dispersion, an emulsion, an elixir, a gel, an ointment, a cream, a patch or a suppository.

The pharmaceutical preparations of the invention can be prepared by known dissolving, mixing, granulating, extrusion, coextrusion or tablet-forming processes. For oral administration, the active ingredients, or their physiologically tolerated derivatives in another embodiment, such as salts, esters, N-oxides, and the like are mixed with additives customary for this purpose, such as vehicles, stabilizers, or inert diluents, and converted by customary methods into suitable forms for administration, such as tablets, coated tablets, hard or soft gelatin capsules, aqueous, alcoholic or oily solutions. Examples of suitable inert vehicles are conventional tablet bases such as lactose, sucrose, or cornstarch in combination with binders such as acacia, cornstarch, gelatin, with disintegrating agents such as cornstarch, potato starch, alginic acid, or with a lubricant such as stearic acid or magnesium stearate.

In addition, the composition can contain minor amounts of auxiliary substances such as wetting or emulsifying agents, pH buffering agents which enhance the effectiveness of the active ingredient.

An active component can be formulated into the composition as neutralized pharmaceutically acceptable salt forms. Pharmaceutically acceptable salts include the acid addition salts (formed with the free amino groups of the polypeptide or antibody molecule), which are formed with inorganic acids such as, for example, hydrochloric or phosphoric acids, or such organic acids as acetic, oxalic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The compositions of the present invention are formulated in one embodiment for oral delivery, wherein the active compounds may be incorporated with excipients and used in the form of ingestible tablets, buccal tables, troches, capsules, elixirs, suspensions, syrups, wafers, and the like. The tablets, troches, pills, capsules and the like may also contain the following: a binder, as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. Syrup of elixir may contain the active compound sucrose as a sweetening agent methyl and propylparabens as preservatives, a dye and flavoring, such as cherry or orange flavor. In addition, the active compounds may be incorporated into sustained-release, pulsed release, controlled release or postponed release preparations and formulations.

Such compositions are in one embodiment liquids or lyophilized or otherwise dried formulations and include diluents of various buffer content (e.g., Tris-HCl., acetate, phosphate), pH and ionic strength, additives such as albumin or gelatin to prevent absorption to surfaces, detergents (e.g., Tween 20, Tween 80, Pluronic F68, bile acid salts), solubilizing agents (e.g., glycerol, polyethylene glycerol), anti-oxidants (e.g., ascorbic acid, sodium metabisulfite), preservatives (e.g., Thimerosal, benzyl alcohol, parabens), bulking substances or tonicity modifiers (e.g., lactose, mannitol), covalent attachment of polymers such as polyethylene glycol to the protein, complexation with metal ions, or incorporation of the material into or onto particulate preparations of polymeric compounds such as polylactic acid, polglycolic acid, hydrogels, etc., or onto liposomes, microemulsions, micelles, unilamellar or multilamellar vesicles, erythrocyte ghosts, or spheroplasts. Such compositions will influence the physical state, solubility, stability, rate of *in vivo* release, and rate of *in vivo* clearance. Controlled or sustained release compositions include formulation in lipophilic depots (e.g., fatty acids, waxes, oils). Also comprehended by the invention are particulate compositions coated with polymers (e.g., poloxamers or poloxamines).

In another embodiment, the compositions of this invention comprise one or more, pharmaceutically acceptable carrier materials.

In one embodiment, the carriers for use within such compositions are biocompatible, and in another embodiment, biodegradable. In other embodiments, the formulation may provide a relatively constant level of release of one active component. In other embodiments, however, a more rapid rate of release immediately upon administration may be desired. In other embodiments, release of active compounds may be event-triggered. The events triggering the release of the active compounds may be the same in one embodiment, or different in another embodiment. Events triggering the release of the active components may be exposure to moisture in one embodiment, lower pH in another embodiment, or temperature threshold in another embodiment. The formulation of such compositions is well within the level of ordinary skill in the art using known techniques. Illustrative carriers useful in this regard include microparticles of poly(lactide-co-glycolide), polyacrylate, latex, starch, cellulose, dextran and the like. Other illustrative postponed-release carriers include supramolecular biovectors, which comprise a non-liquid hydrophilic core (e.g., a cross-linked polysaccharide or oligosaccharide) and, optionally, an external layer comprising an amphiphilic compound, such as phospholipids. The amount of active compound contained in one embodiment, within a sustained release formulation depends upon the site of administration, the rate and expected duration of release and the nature of the condition to be treated suppressed or inhibited.

In one embodiment, the methods, kits or compositions of the present invention may be used to improve the therapeutic efficiency of a cancer therapy, or treatment of HIV, Herpes simplex, Herpes Zoster, mycobacterial infection, cancer, psychotic disorders, multiple sclerosis, diabetes, kidney disease, chronic pain, Hepatitis or any other applicable disease or disorder, such as, in another embodiment, a chronic disease, where a prolonged therapeutic regimen involving repeated administration of at least one drug is beneficial.

The following examples are presented in order to more fully illustrate the preferred embodiments of the invention. They should in no way be construed, however, as limiting the broad scope of the invention.

### EXAMPLES

### Example 1: Haloperidol Release from PLGA implants in Monkeys

### In-vivo release of haloperidol in monkey:

Studies to evaluate 1 year of continuous haloperidol delivery in primates were performed. Effects of long-term haloperidol exposure on cortical anatomy in primates, as a control for antipsychotic exposure in post mortem human schizophrenia studies were evaluated. The study demonstrated 14 months of haloperidol release in monkeys.

### Subjects:

The Institutional Animal Care and Use Committee at the University of Pittsburgh approved all protocols. Two monkeys (*Macaca fascicularis,* Rangos Research Facility) received implants. One animal received control implants with no drug, while the other received implants with 40% haloperidol by mass. Haloperidol (Sigma, St. Louis, MO) dosing was approximately 1 mg/kg/day over 12 months to achieve a serum concentration of 2-10 ng/ml. Each implant was made from a single polymer of Poly(d,l-lactic-glycolic acid) copolymer (PLGA) in ratios of 75:25, 85:15, 90:10 (high and low inherent viscosity), 95:5 and 100:0 (Medisorb® Aldermes, Cincinnati, OH), resepectively, polylactic acid (PLA):polyglycolic acid (PGA).

### Implant fabrication:

Polymers and haloperidol were mixed in a proportion of 60/40 by mass and solvent cast from acetone (Fisher Scientific, Pittsburgh, PA). The resulting film was compression molded to disk-shaped implants of 20 mm diameter with average thickness of 1.22 ± 0.0 mm, mass 493 ± 2 mg and density of 1.28 ± 0.0 g/cc.

### Pharmacokinetic determination:

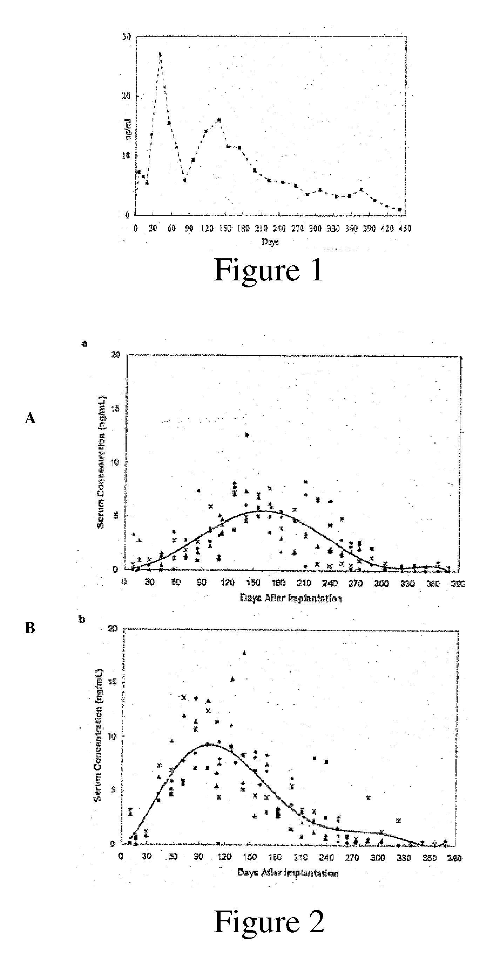

Blood was drawn twice per month. Specimens were separated by centrifugation and haloperidol levels assayed were done by high-pressure liquid chromatography (HPLC) with ultraviolet (UV) detection (Figure 1). Assays from control animals yielded no drug detected.

### Results:

As depicted graphically in Figure 1, haloperidol release was measured over a total of 443 days. Average serum concentration during the first 224 days is 10.5 ±1.5 ng/ml. During the subsequent 176 days, serum haloperidole level is sustained at lower concentration befor the end of release. Mean concentration during this period is 4.0 ±0.4 ng/ml. During the last 45 days, release follows 1^{st} order decay release with a mean serum level of 1.2±0.3 ng/ml.

### Example 2: Release of Haloperidol from two-polymer PLGA system in Rabbit implants

### Haloperidol release in Rabbit:

Implants in rabbits were tethered to assist in locating implant sites at necropsy. Two different implant systems were tested. One contained an amalgam of five different polymers to affirm previous findings in monkey (see example 1). The other was composed of a single long-lasting polymer system, aimed at reducing the initial spike while maintaining release for one year. These studies demonstrated haloperidol release for 13 months in rabbit.

### Subjects:

Rabbits (N=12, Covance, Denver, PA) ranged from 4.0 to 5.7 kg. Five animals received implants comprising a single polymer, 100% Poly(Lactic Acid) (PLA), with a 40% (w/w) haloperidol load for an average dose of 418±7 mg/kg yielding a daily dose of 1.13±0.02 mg/kg/day for anticipated delivery over 365 days. Five additional animals received implants comprising a combined polymer system including 75:25, 85:15, 90:10 high inherent viscosity, 90:10 low inherent viscosity and 100:0 PLGA. The average dose in this group was 473±4 mg/kg with an expected delivery over 365 days yielding an average daily dose of 1.29±0.03 mg/kg/day. Two rabbits received implants without drug as a control. One control received 100% PLA implants to mimic the single polymer condition, while the other received implants composed of 75:25, 85:15, 90:10 high inherent viscosity, 90:10 low inherent viscosity and 100:0 PLGA to mirror the combined polymer system.

### Implant fabrication:

Implants for rabbits were made using procedures described in example 1 for monkeys with an average mass of 536±2 mg and density of 1.24±0.00 g/cc.

### Pharmacokinetic determination:

Blood samples were drawn twice per month. Serum haloperidol was determined by HPLC with UV detection following solid phase extraction (Oasis MCX columns, Waters, Milford, MA) (Figure 2).

### Histopathology:

Five rabbits were sacrificed after nine months to obtain interim pathological analyses. The remaining seven rabbits were sacrificed after an additional four months of study. Remains of implants were found tethered in place upon sacrifice in all animals except controls. HPLC/UV and NMR spectroscopy confirmed the presence of haloperidol and PLGA breakdown products in residual implants. Histological analyses showed all organ systems in control and treated rabbits were within normal limits.

### Results:

As depicted graphically in Figure 2A, haloperidol release from a single Polymer system in Rabbits over a total of 379 showed average serum concentration during the first 224 days was 2.5 ±0.4 ng/ml. Between days 57 and 274, mean concentration was 3.8±0.4 ng/ml.

As depicted graphically in Figure 2B, haloperidol release from a combination Polymer system in Rabbits over a total of 379 showed average serum concentration during the first 224 days of 2.5 ±0.4 ng/ml. Between days 57 and 274, mean concentration was 3.8±0.4 ng/ml.

In multiple polymer systems design, higher serum levels were apparent in the first seven months in comparison to the subsequent seven months (Fig. 2B). It is likely that implants made from faster-degrading polymers such as 75:25 PLGA contributed to a larger initial phase of release. Therefore, the mass of 75:25 PLGA implants could be reduced in future studies to achieve lower initial dosing.

### Example 3: Release of Risperidone from PLGA implants

### Risperidone Stability:

Risperidone (10 mg) was dissolved in 100 µl of Acetonitrile for subsequent dissolution in 1000 ml of phosphate buffered saline, pH 7.0 to yield a final solution of 10,000 ng/ml. Risperidone solution was then stored in a light-safe amber bottle at 37 °C and shaken at 40 rpm. 1 ml samples were taken 3 times per week and examined by UV spectroscopy (Amersham Biosciences, Buckinghamshire, UK) for drug content. Analysis indicated that the concentration of drug remained stable with an overall decrement of 0.12% over 285 days, equivalent to 0.05 ng/day.

### In-vitro Risperidone release from individual polymers:

Risperidone release profile from three polymers were evaluated. Implants were fabricated as described in examples 1 above, with 20% Risperidone (w/w)
(RBI Flanders, NJ) and 80% polymer (MEDISORB®, Alkermes Inc. Cincinati, OH), where the polymer consisted of 50:50 (PLA: PGA) High inherent viscosity (IV), 65:35 Low IV and 75:25 High IV PLGA . Three implants were placed in a separate light-safe amber bottle (500 ml) of phosphate buffer saline at 37 °C and shaken at 40 rpm. 1 ml aliquotes were taken 3 times per week from each bottle and analyzed by UV spectroscopy (Amersham Biosciences, Buckinghamshire, UK) for drug content, after which 1 ml of buffer was reintroduced to maintain constant volume.

### Results:

As depicted graphically in Figure 3, cumulative Risperidone release from implants containing either 50:50 High IV, 65:35 Low IV and 75:25 High IV PLGA with 20% drug load, expressed as total mass of Risperidone released, follow a typical saturation curve. Drug release is a function of the fractional concentration of PGA in the polymer matrix, wherein the lower the fractional concentration of PGA, the slower the release of Risperidone.

### Example 4: Determination of maximum load of Risperidone in the implants

### Determination of maximum Risperidone drug Load

Studies examining the effect of drug concentration on Risperidone release profile are done with the purpose of establishing the maximum concentration of Risperidone that can be used in implants. Maximum drug loading per implant system is used to minimize implant system size, thereby making it more tolerable.

### Methodology:

Implants are prepared using a single polymer (85:15 PLGA High IV) combined with Risperidone at ratios of 20, 40 or 60% drug (w/w). The polymer has an anticipated degradation time of 4 months. Each implant has a mass of 50 ±10 mg yielding drug mass of 10, 20 and 30 mg respectively. Representative release profile expressed as total mg Risperidone from each 50 mg implant, over 40 days is depicted graphically in Figure 4A. Representative release profile expressed as precentage of the amount of Risperidone in each 50 mg implant, over 40 days is depicted graphically in Figure 4B.

### Example 5: Release of Haloperidol from PLGA implants as a function of implant geometry

### Background:

The large incision associated with disk-shaped implants may reduce their applicability to human subjects, under some circumstances. Therefore, implant fabrication was modified to produce rod-shaped implants that can be inserted through a 4 mm hole. Rigid PLGA implants obviate the need for a tool to guide the implants under the skin, to ensure subcutaneous, rather than intramuscular insertion. Preliminary studies were then carried out to examine the effect of implant geometry on haloperidol release.

### Methodology:

Haloperidol (40% w/w) was combined with 50:50 PLGA (60% w/w) by solvent casting. Material was then compression molded into discs or slowly extruded using high pressure piston extruder (DACA Instruments, Goleta, CA).

### Results:

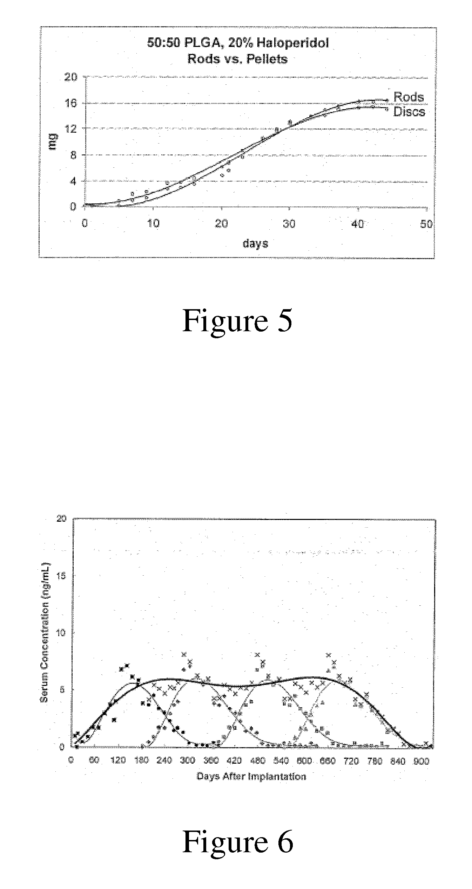

Release profiles from these two geometries are shown in Figure 5. As depicted graphically, Figure 5 shows the cumulative *in-vitro* release from disk and rod-shaped implants. Each point represents the mean of triplicate observations of disks or rods. Each implant was disposed in 500 ml of phosphate buffered saline, pH 7.0 at 37 °C at 40 RPM in the dark. Rods and disks were matched for weight. No appreciable differences in release profile were found, between the two implant geometries evaluated under these conditions.

### Example 6: Control of release profiles to maintain consistent serum drug level

### Gaussian Release profiles:

Release profiles from the single-polymer system in rabbit (Figure 2A) suggest that biodegradable implants can deliver antipsychotic medication for an extended therapy period. While this design avoids early periods of high serum concentration, the Gaussian pattern of release results in a 3-month lag to achieve target serum concentration. Nevertheless, the symmetrical nature of the release profile opens the possibility of using overlapping implantations every six months to sustain drug delivery throughout the period of therapy (Figure 6) thereby offsetting the steady decline from one set of implants with the gradual onset from a subsequent set.

Figure 6 shows a model for continuous delivery from biodegradable implants. The release profile from 100% poly(lactic Acid) (PLA) implants in rabbits is superimposed temporarily at 6 months intervals (gray trendlines) to model reimplntation intervals. Projected serum levels are summed at each time point, resulting in an anticipated total release plot (Xs). The black trendlines model total release data. The system oscillates around a target serum level as long as reimplantations occur every 6 months.

### Example 7: Scaling of sustained release drug therapy to human patients

### Scaling to Human:

The substantial interspecies differences in the metabolism of many drugs, including haloperidol, with both rabbits and monkeys requiring approximately 15 to 30 fold higher doses than humans for equivalent plasma concentrations are considered (Bacopoulos et al., 1980; Jibiki et al., 1993; Klintenberg et al., 2002). Humans require approximately 1 mg/kg/month of haloperidol when given as a depot preparation, an implant system containing 600 mg provides one year of treatment for a 50 kg patient. Thus, implant design has 1.5 gm of material with 40% drug load for one year of medication. High potency agents such as Risperidone showing promise for this approach are used as well. Release profiles for Risperidone indicate that an in-vivo system can attain long-term delivery for six months or more when incorporated to a PLGA delivery system.

### Example 8: Long term release of Risperidone

### Physico-chemical characteristics of Risperidone in long-term delivery system: Stability of Risperidone

The stability of Risperidone is evaluated following solvent casting from acetone and slow extrusion molding, thereby ensuring that the compounds can withstand commercial processing into implants. Subsequently, a shelf-life study is conducted wherin Risperidone content is examined in a series of implants stored at 4°C for various intervals up to 1 year. Risperidone is stable in a physiologic aqueous environment (37 °C, phosphate bufferred 0.9% saline, pH 7.0) for over 10 months when examined with UV spectroscopy. Risperidon retains its characteristic HPLC-MS peak retention time and mass, following solvent casting and compression molding. In addition, Risperidone displays less than 1% degradation during 1 year storage and similarly, Risperidone displays less than 1% degradation during 1 year storage in a physiologic aqueous environment (37 °C, phosphate bufferred 0.9% saline, pH 7.0) as assessed by quantitative HPLC equipped with UV detector.

### Stability of PLGA copolymers:

The stability of ten (10) PLGA polymers during fabrication storage is evaluated by measuring inherent viscosity and glass transition temperature (Tg). PLGA polymers display less than 5% change in inherent viscosity and less than 2°C change in Tg following solvent casting and extrusion molding in a dry environment at 4°c for 1 year.

### Methods:

### Materials

Risperidone is synthesized using GMP methods. Commercially available, medical-grade Risperidone and acetone (USP/EP) is used

The following PLGA polymers are used:

| No. | PLGA Type PLA:PGA (IV) | Tg (°C) | Degradation Interval (Months) |
|---|---|---|---|
| 1 | 50:50 (L) | 50-55 | 1-2 |
| 2 | 50:50 (H) | 50-55 | 1-2 |
| 3 | 65:35 (L) | 45-50 | 3-4 |
| 4 | 65:35 (H) | 45-50 | 3-4 |
| 5 | 75:25 (L) | 48-53 | 4-5 |
| 6 | 75:25 (H) | 48-53 | 4-5 |
| 7 | 85:15 (L) | 50-55 | 5-6 |
| 8 | 85:15 (H) | 50-55 | 5-6 |
| 9 | 100:0 (L) | 50-55 | 12-16 |
| 10 | 100:0 (H) | 50-55 | 12-16 |

### Temporal stability of Risperidone in physiologic solution:

Three replicates of Risperidone solution is prepared as follows: Risperidone (10mg) is dissolved in 100 µl of Acetonitrile for subsequent dissolution in 1000 ml of phosphate buffered saline, pH 7.0 to yield a final solution of 10,000 ng/ml. Risperidone solution is then stored in a light-safe amber bottle at 37 °C. Three aliquots (100µl) of each sample will be drawn at monthly intervals for HPLC analysis. The concentration of Risperidone is then plotted against time and the slope of the resulting line reflects the stability of the compound.

### Stability of Risperidone and PLGA polymers during implantation:

### Risperidone:

Risperidone implants composed of 5% drug and 95% polymer (w/w) are made by solvent casting from acetone followed by slow extrusion molding into rods measuring 3.6mm in diameter and 20 ± 10 mm length at 60°C and 5mm/sec piston speed. The resulting material undergoes quantitative *in vitro* analysis with HPLC for Risperidone content. The resulting measurements are expressed as as a ratio of the original mass of Risperidone in the implant material.

### Polymers:

Stability of PLGA polymers is assessed following fabrication procedures. Control implants are prepared without drugs, using solvent casting from acetone followed by slow extrusion molding. The resulting material (5-10 mg) undergoes analysis for alterations in IV in acetone, using an Ubbelhode suspended-level viscometer at constant temperature (25 °C) and changes in Tg using diferential scanning calorimetry between 20-100°C at 10 °/min. Both IV and Tg are determined pre and post implant fabrication

### Determining in-vivo release profile for Risperidone as a function of biodegradable copolymer physico-chemical properties:

### Release patterns for individual polymers:

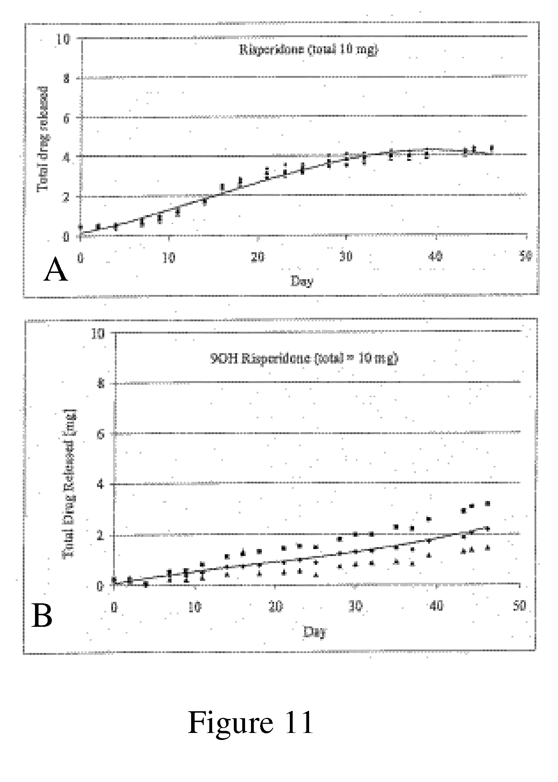

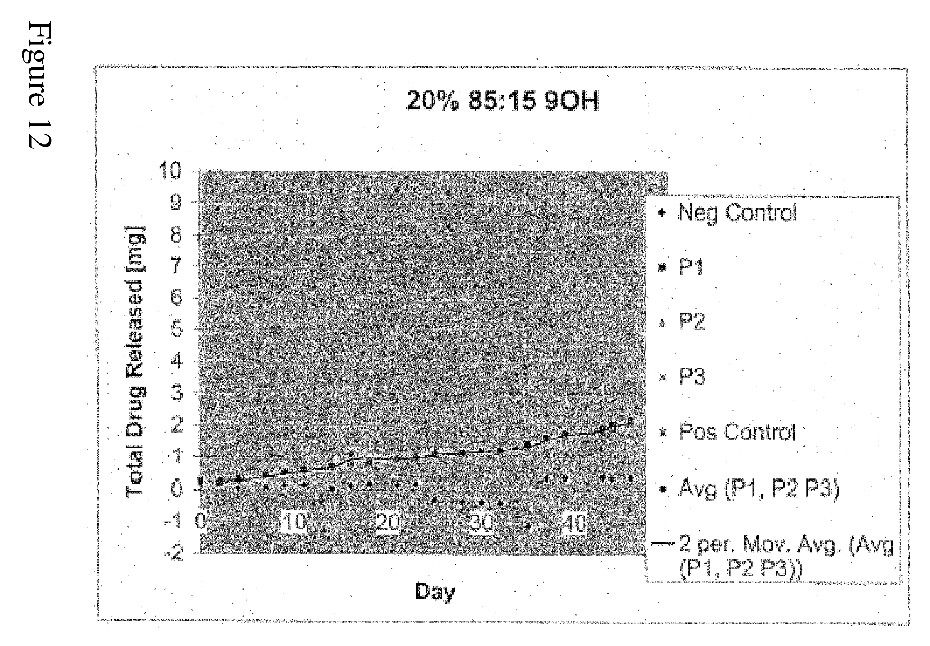

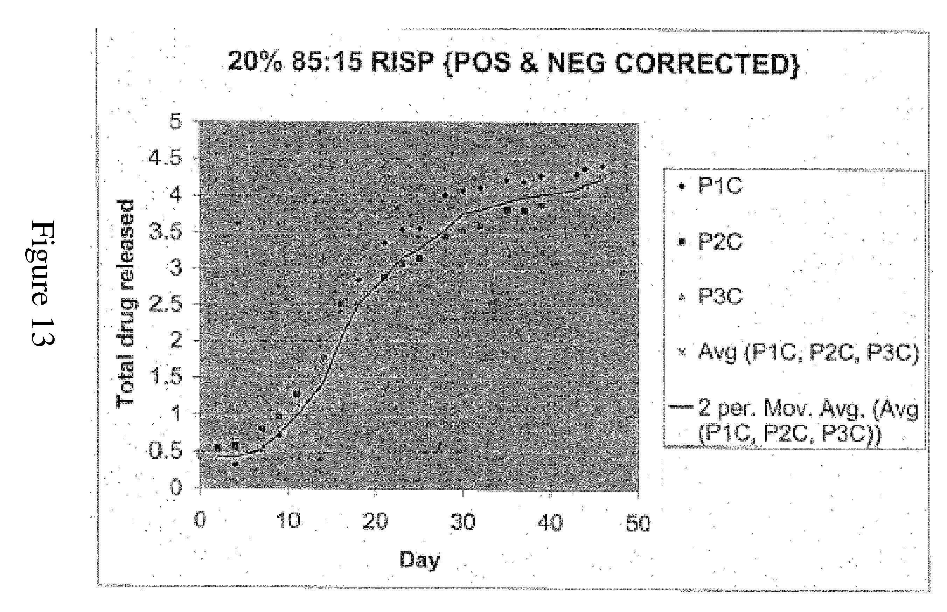

Single polymer systems containing 100% PLA and 50% drug load result in Gaussian release profile, with peak concentration at 6 months and within therapeutic range (above 1 ng/L) between 2-8 months post-implantation (Figure 7A). The symmetrical nature of the release profile opens the possibility of using overlapping implantations every six months to sustain drug delivery throughout the therapy period. Such a system provides sustained therapeutic drug delivery because each reimplantation is offsetting the steady decline from one set of implants with the gradual onset of release from a subsequent set. Typical release of Risperidone is shown in Figure 11A depicting cumulative release of Risperidone dispersed in PLGA biodegradable matrix. Similar profile is shown for 9_OH-Risperidone in Figure 11B, similarly, Figure 12 shows the cumulative release of 20% (w/w) 9-OH-Risperidone dispersed in 85:15 PLA:PGA block copolymer biodegradable matrix. Figure 13 shows the graph of cumulative release of 20% (w/w) Risperidone dispersed in 85:15 PLA:PGA block copolymer biodegradable matrix.

To avoid relatively extensive initial lag time and high-amplitude oscillation around the desired therapeutic concentration, additional polymer implants or fast release formulations are combined and their implantation is staggered throughout the therapy period yielding a pseudo—zero-order release wherein therapeutic drug levels are sustained throughout the therapy period (Figures 7B-7E).

### Example 9: Release of Haloperidol from PLGA implants

### Implant fabrication:

Implants were fabricated through solvent casting and compression-molding. Two polymers, 75% polylactide with 25% polyglycolide (75:25 PLGA) and 85% polylactide with 15% polyglycolide (85:15 PLGA) were presented in a combined system of release during a 5-month period. Each copolymer had a distinctive period of degradation that was determined by the ratio of lactide to glycolide and the molecular weight of the resulting molecule produced. An additional polymer of 100% polylactide (PLA) was used for in vivo testing in rats. All polymers (Alkermes Inc., Cincinnati, OH) had an inherent viscosity of 0.66 - 0.80 DL/g in chloroform and a molecular weight distribution between 120-140 kD. Individual polymers and haloperidol (Sigma, St. Louis, MO) were dissolved in acetone and solvent cast at 60 °C for 72 hours. Solvent-cast material was compression molded at 80 °C and 25,000 psi to a final density of 1.1 ±0.05 g/cc)

### In-vitro assay:

Individual implants were placed in 1 liter of phosphate buffered saline (PBS), pH 7.0 at 37°C on a shaker. Haloperidol concentration was measured by GCMS (National Medical Services, Willow Grove, PA). Each assay included negative controls of implants made of polymer alone and a 100 ng/ml haloperidol standard to assess stability of haloperidol in solution over time.

### Animals

Implants were tested in rats (Harlan, Indianapolis, IN) (n=9) and mice (Jackson Labs, Bar Harbor, ME)(n = 16). All animals were housed in an AAALAC accredited animal facility at the University of Pennsylvania. The Institutional Animal Care and Use Committee (IACUC) approved all protocols. Animals were maintained with a 12:12 light:dark cycle with all testing and procedures performed during the light cycle.

### Implantation/removal surgery:

Mice and rats were anesthetized with ketamine/xylazine (100/10 mg/kg, i.p.). A 1-cm scalpel incision was made in the skin on the dorsal aspect of the animal. The subdermal space was visualized with hemostats and a single implant was placed between dermis and muscle with forceps. The wound was then closed with a surgical staple. Four weeks after implantation, implants were localized by palpation and removal was performed with identical anesthesia and incision. In-tact implants were easily removed with forceps.

### Behavioral testing:

Sixteen C57bl/6 mice received implants made of 75:25 PLGA alone (n = 8) or 75:25 with 20% haloperidol (n = 8) to assess the effects of implants on locomotion. Following three weeks of implantation, total distance traversed was assessed over a twenty-minute period. Implants were then removed and animals allowed to recover for 48 hours prior to re-testing 20 minutes after apomorphine challenge (0.5 mg/kg i.p.) (Sigma, St Louis, MO).

### Western blot:

Six Sprague Dawley rats received implants made of PLA with 30-40% haloperidol. Three rats received implants of PLA alone. Implants remained in all animals for three months prior to removal. Seventy-two hours after implant removal, rats were sacrificed and brains rapidly removed, dissected into four regions (cortex, hippocampus, striatum and cerebellum) then frozen in liquid nitrogen. Western blots for quantitative analysis of D2 receptor protein were performed on striatum. Three concentrations of cortex protein from a single animal (2.5, 5 and 10 µg) were run with all blots as an internal control to insure intensity of labeling was within linearity of quantitative software. Only those blots in which the density of samples was within the linear range of internal standards were used for analyses. Western blots were performed using polyclonal antibody WR-3526, raised against amino acids 272-282 of the D2 receptor protein (Research and Diagnostic Antibodies, Berkeley, Ca). Striata were homogenized in homogenization buffer (20 mM HEPES, 2mM EGTA, 1 mM PMSF, 2 µM Aprotinin and 2mM DTE), followed by a 30-second sonication. Samples were centrifuged 100,000 g for 1 hour at 4°C. Pellets were resuspended and solubilized in homogenization buffer containing 0.1% Triton X-100. Proteins were extracted on ice for 45 minutes with occasional agitation. After extraction, proteins were centrifuged at 30,000 g for 30 minutes at 4°C. Protein samples were prepared with 25% 4x NuPAGE sample buffer plus 10% reducing agent (Invitrogen) and heat shocked at 70°C for 10 minutes. Samples were separated on a 10% precasted mini-gel at 200 volts for 50 min. Proteins were transferred to PVDF at 30 volts for 1 hour. Blots were blocked with 5% milk in TBS (20 mM Tris, pH = 7.5, 0.5 M NaCl), then washed for 15 minutes. Blots were then incubated overnight with anti-D2-Receptor antibody, washed with TBS, and incubated with goat anti rabbit horseradish peroxidase conjugate (BioRad, 1:4800) for 1 hour. Blots were then incubated with chemiluminescent substrate (Pierce) for 1 min, wrapped with plastic and exposed to autoradiographic film.

### Quantification:

The intensity of each band was quantified using a densitometer model 7100 and quantitative analysis software (Bio Rad, Hercules, CA) and expressed as a ratio to the corresponding band in rat 1 to yield a ratio of intensity (rat 1 = ratio of 1). All samples were processed simultaneously on a single blot to allow for quantitative comparisons between conditions.

### In-vitro:

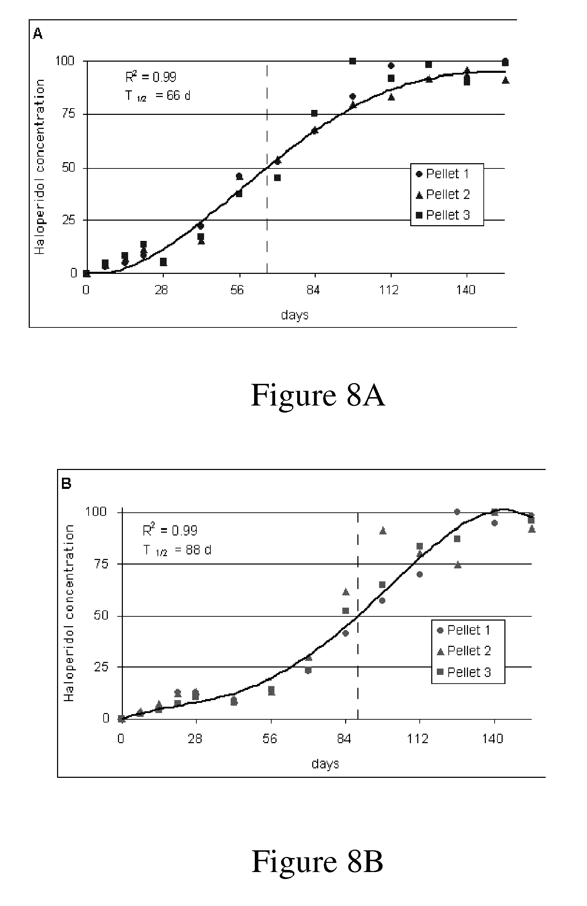

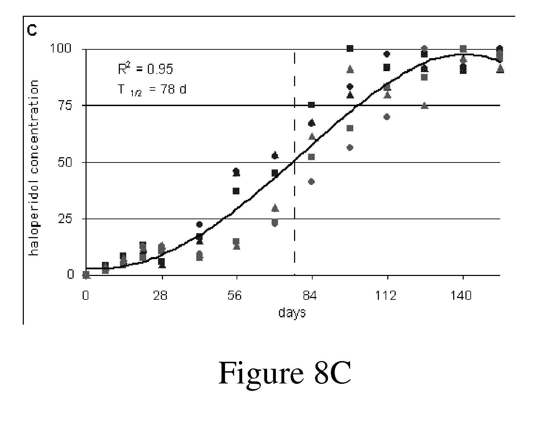

Haloperidol concentration for two polymers, as well as combined values are shown in Figure 8A-C. Figure 8 graphically depicts individual implants that are placed in phosphate buffered saline, pH 7, at 37 °C on a shaker. Samples of buffer are drawn at weekly / biweekly intervals and evaluated with GCMS for haloperidol concentration. Cumulative release is expressed as the percent of total drug load measured in buffer solution at each time point. Cumulative haloperidol concentration from 75:25 and 85:15 PLGA polymers. Each graph displays data from three implants (circle, square and triangle) with a 4th degree polynomial average line for the three values.

Implants made of 75:25 PLGA with 40% haloperidol resulted in a pattern of release characterized by an initial phase of slow release (approximately 0.29% / day / implant) from 0 to 28 days. A second phase of more rapid release occurred between 28 and 84 days (approximately 1.28% / day / implant). Implants made with 75:25 PLGA released 50% of the total haloperidol load in 66 days (Figure 8A). Implants made of 85:15 PLGA with 40% haloperidol displayed a similar pattern of release with a phase of slow release (approximately 0.26% / day / implant) from 0 to 56 days. A second phase of more rapid release occurred between 56 and 140 days (0.95% / day / implant). Implants made from 85:15 PLGA released half of the haloperidol load in 88 days (Figure 8B). Release from the theoretical composite system of 75:25 and 85:15 PLGA, 40% haloperidol, shows an early phase from 0 to 28 days with an average of 0.34%/day and more rapid release between 28 to 140 days with 0.82%/day. The combined system of 75:25 and 85:15 PLGA demonstrates a period of 78 days to release half of the total haloperidol load (Figure 8C). The value for the positive control solution (100 ng/ml) remained stable throughout the period of 154 days (0 days = 106, 154 days = 100, mean ± sd throughout study interval = 106 ±11 ng/ml).

### In-vivo Locomotor activity:

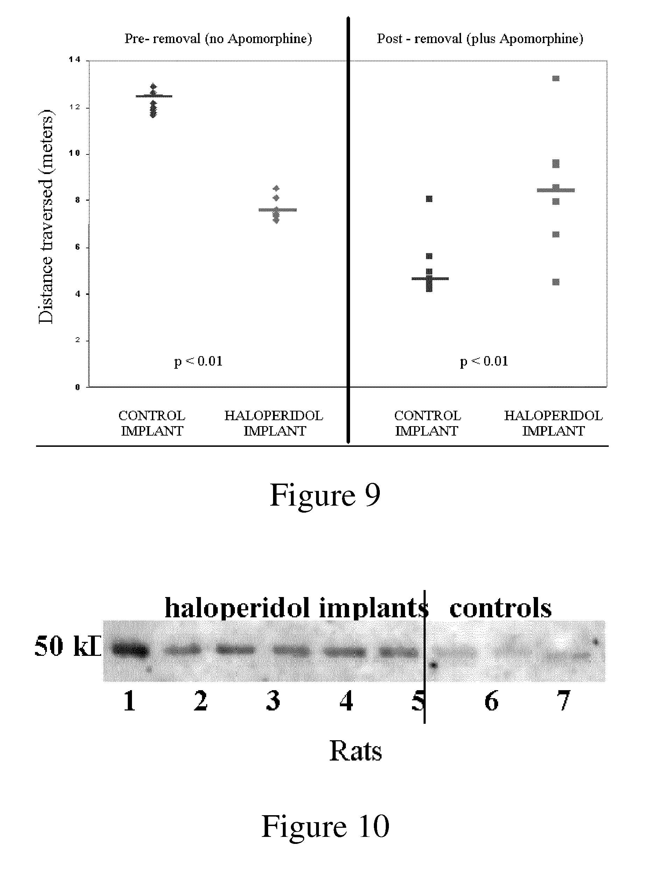

Locomotor activity for mice that received either haloperidol or blank polymer implants is demonstrated in Figure 9. All animals were tested 3 weeks after receiving implants made of 75:25 PLGA alone or 75:25 PLGA with 20% haloperidol. Baseline locomotor activity was measured for twenty minutes. Animals with control implants traveled a mean of 12223 ± 433 cm, while those with haloperidol containing implants traveled an average of 7664 ± 450 cm. Thus, mice with haloperidol implants traveled significantly less distance than controls (p < 0.01). Implants were then removed and all animals were allowed to recover for 48 hours. One animal with a control implant died from anesthetic during removal surgery. Forty-eight hours after removal of implants, animals received apomorphine 0.5 mg/kg i.p. twenty minute prior to locomotor testing, which has been shown to increase locomotor activity in mice (Ninan and Kulkarni 1999) . After apomorphine challenge, animals that had control implants traveled a mean of 4721 ± 476 cm, while those with haloperidol containing implants traveled an average of 8531 ± 2536 cm. Therefore, following removal of implants and exposure to apomorphine, mice that had haloperidol implants traveled more distance than control mice (p<0.01).

### Western blots:

Western blots of striatal membranes from all rats revealed a band at an apparent molecular weight of approximately 50 kD (Figure 10) corresponding to the predicted molecular weight of the full-length D2 receptor protein (Expert Protein Analysis System, Swiss Institute of Bioinformatics, http://www.expasy.ch/) (Bunzow et al 1988) . Mean optical density of bands was quantified relative to the corresponding band in lane 1 (haloperidol treated rat). Results based on 3 blots yielded a mean ± SD relative density for haloperidol implant treated rats of 0.90 ± 0.07 for the 50 kD band, while the mean relative density for control rats was 0.64 ± 0.02 (p<0.001, two tail t-test).

### Example 10. Risperidone Implant Long-term Therapy for Scizopherenia

### Implant Fabrication:

### Risperidone Disk implant

Implants may be fabricated through solvent casting and/or compression-molding. Two polymers, 75% polylactide with 25% polyglycolide (75:25 PLGA) and 85% polylactide with 15% polyglycolide (85:15 PLGA) are presented in a combined system of release during a 12-14-month period. Each copolymer has a distinctive period of degradation that is determined by the ratio of lactide to glycolide and the molecular weight of the resulting molecule produced. All polymers (Alkermes Inc., Cincinnati, OH) are with an inherent viscosity of 0.66 0.80 DUg in chloroform and a molecular weight distribution between 120,000-140,000. Individual polymers and Risperidone (Sigma, St. Louis, MO) may be dissolved in acetone and solvent cast at 60 °C for 72 hrs. Solvent-cast material was made into disks measuring 20 mm in diameter and 1.2 ± 0.00 mm thickness, with final density of 1.2 ± 0.1 g/cc.

### Risperidone Rod implant

Rod Implants may be fabricated through compression-molding. PLGA polymer, 50% polylactide with 50% polyglycolide (50:50 PLGA) are presented in a system of release during a 2-month period. Polymer (Alkermes Inc., Cincinnati, OH) is with an inherent viscosity of 0.66±0.1 DL/g in chloroform and a molecular weight distribution between 120,000-140,000. Polymer and Risperidone (Sigma, St. Louis, MO) are dissolved in acetone at a 95:5 ratio (w/w) and extruded using high pressure piston extruder (DACA Instruments, Goleta, CA) at 60°C and 5mm/sec piston speed psi to a final density of 1.1 ± 0.1 g/cc. And 3.5 ± 0.5 mm diameter and 20 ± 10 mm length. Release profile may be altered by varying monomer ratio (lactide:glycolide), initial drug loading and fabrication procedures.

### Experimental Controls and Monitoring:

A) The first group of control subjects receive 6 months of risperidone exposure from a multiple polymer system.
B) The second group of control subjects receive an initial multi-polymer system followed by reimplantation of "maintenance" implants at six months to provide 12 months of continuous medication prior to necropsy.
C) The third group of control subjects receive an initial multi-polymer system to provide six months of steady state delivery, but will not receive reimplantation of maintenance implants. These control subjects are then be tested at 12 months post implantation to provide toxicological evaluation after the system has ceased to delivery medication.
D) The fourth group of control subjects receive broken implants to evaluate the consequences of mechanical damage to implants during the delivery interval.
E) The fifth group of control subjects serve as positive control and they receive daily oral risperidone or risperidone injections to reveal toxicological effects of the drug that are independent of the delivery system being tested.
F) The final group of control subjects receive blank implants with no drug as a negative control to assess the effects of polymer materials, independent of risperidone.

### Rod Implant Disposition

Implants varying in release profiles are inserted subcutanously to a patient in need thereof. The implans may be disposed at the shoulder through a 4 mm incision. Since the implants are rigid, no implant-guides are necessary.

### Determination of therapeutic effect in Schizopherenia:

Patients, while undrgoing therapy, have improved Brief Psychiatric Rating Scale (BPRS) scores (Dinakar, H.S. et al., [2002] Efficacy of Olanzapine and Risperidone for Treatment-Refractory Schizophrenia Among Long-Stay State Hospital Patients, Psychiatric Services, Vol. 53 No. 6, pp 755-757) and show no significant side effects such as weight gain. Improvement is also observed with regards to negative symptoms (alogia, affective flattening, avolition, apathy, social withdrawal and attentional impairment) and performance of cognitive tasks (Honey, G.D., et al., [1999] Differences in Frontal Cortical Activation by a Working Memory task after Substitution of Risperidone for Typical Antipsychotic Drugs in Patients with Schizophrenia. PNAS, vol. 96, No. 23, pp. 13432-13437).

### Blood Serum Levels of Risperidone:

2 ml of blood are taken from the subject every three months or as needed based on the implantation/reimplantation regimen. Blood is then centrifuged and the serum separated. Risperidone concentration is determined following solid-state extraction (MCX, Waters) and HPLC/UV detection.

### Example 11. Microparticles for Oral-delivery of 9-OH-Risperidone for bipolar disorder, short term therapy (1-3 weeks).

### Microparticles fabrication:

The microparticles are made from poly(glycolic acid) and poly(d,1-lactic acid) copolymer at a 75:25 ratio of lactide to glycolide with 9-hydroxy-Risperidone, as an active agent at 98:2 w/w ratio. PLGA copolymer and 9-hydroxy-Risperidone are dissolved in acetone and EVA as the dispersed phase. The continuous phase is made with PVA, water, ethyl acetate, and benzyl alcohol. The dispersed and continuous phases at a 20:80 ratio are pumped through a static mixer (Kenics^{®} KM Series Static Chemineer, Dayton, OH) to form an emulsion. Final microsphere size may be controlled by phase ratio, flowrate, temperature and continuous phase composition. The resulting emulsion is passed into a quenching liquid. After being allowed to settle, the resulting microspheres are then filtered and washed repeatedly with a series of appropriate solvents and then dried.

### Administration of microspheres:

Microspheres are incoprorated in capsules and orally administered to a patient in need thereof. The microspheres may be incorporated with excipients and used as ingestible capsules, which also contain a binder, excipients, a lubricant and possibly a liquid carrier.

### Treatment efficiency:

Patients undergoing long-term therapy exhibit improvement of at least 7 points above baseline based on Clinical Global Impression (CGI), and mean Global Assessment of Functioning (GAF) scores. Treatment is well tolerated, and no patient experiences worsening of mood symptoms while receiving 9-OH-Risperidone (Ghaemi, S.N. [1997] Acute Treatment of Bipolar Disorder with Adjunctive Risperidone in Outpatients. Can J Psychiatry, Vol 42,pp. 196-199.

### Example 12: Sustained release therapy of psychotic disorder using second generation antipsychotic agent:

### Starter formulation:

To compensate for the relatively long lag time associated with attaining therapeutic blood-serum level in rigid implants, a patient exhibiting symptoms of psychotic disorder is given a formulation containing the antipsychotic drug in the form of an injectible for practically immediate elevation of blood serum level to above therapeutic levels. The injectible formulation is followed by oral dose of microparticles of PLGA containing the antipsychotic drug, wherein fast release of the drug, to above-therapeutic levels is obtained in a day and peak delivery is obtained over a two-week period.

### Implants:

### Single polymer implant:

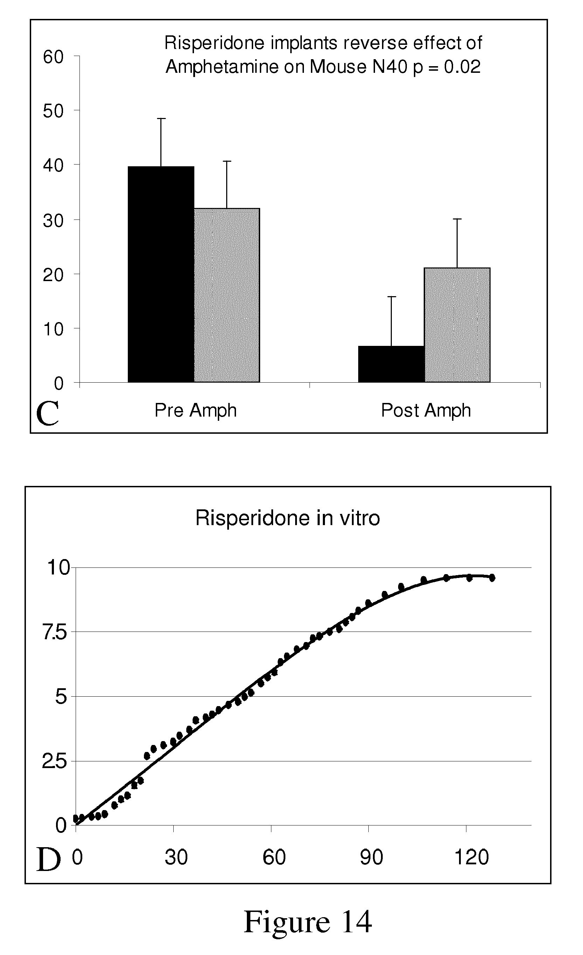

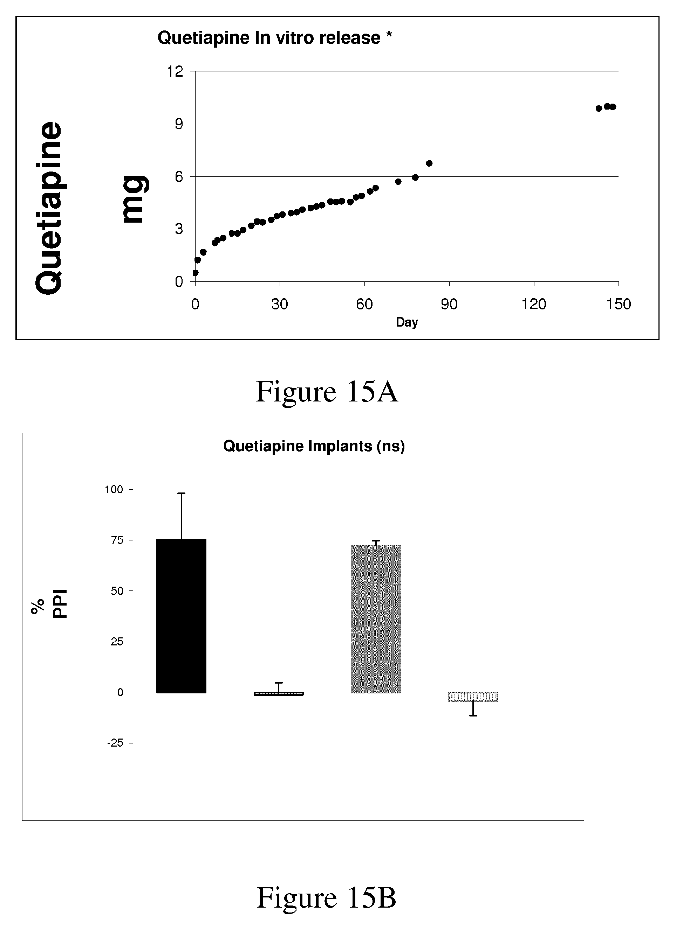

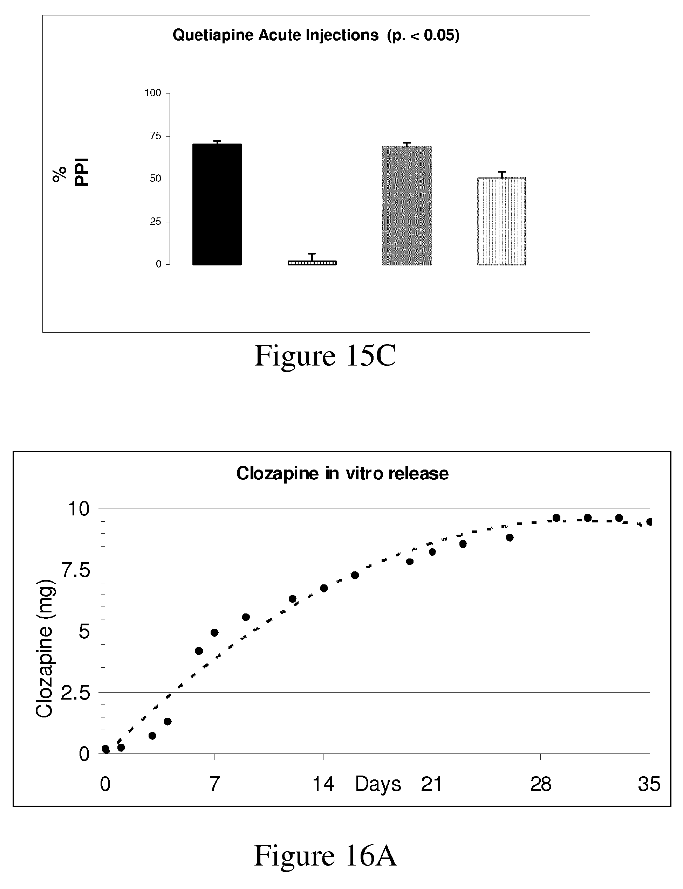

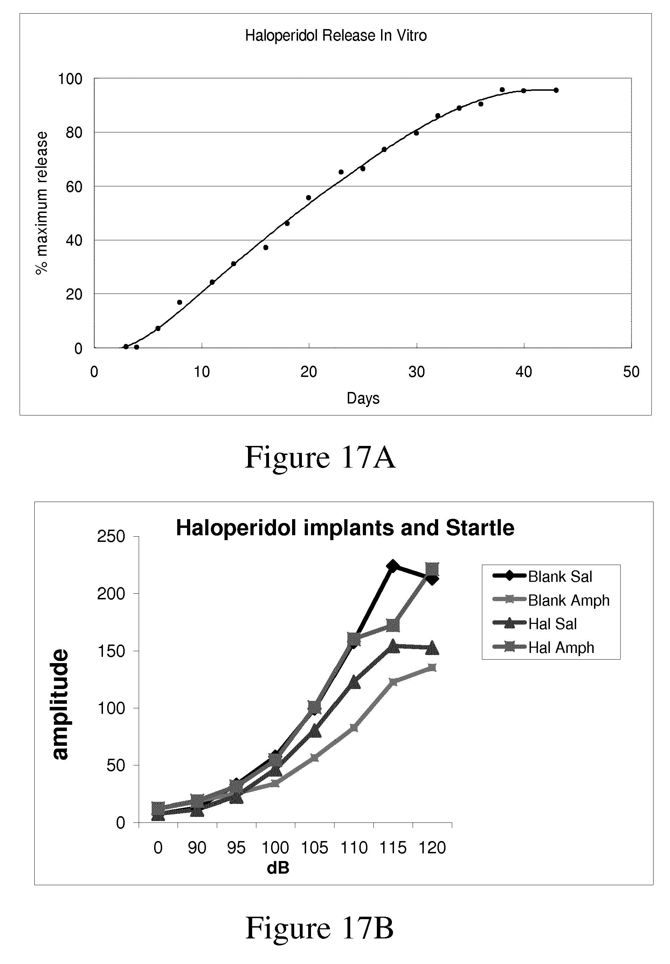

Implants fabricated as described above are made from a single copolymer containing the antipsychotic agent. Release profile is designed to achieve therapeutic levels within 3 weeks of implantation and last from 6-8 months until full degradation, with peak release occurring 5-7 months following implantation. In order to compensate for the high amplitude oscillation around optimal serum level of the antipsychotic agent that may result from a single implant, a second maintenance set of a single copolymer composition may be added, with peak delivery at 10- 14 weeks and full degradation within 4-5 months from implantation. Examples of cumulative release profile for several antipsychotic agents from PLGA implants are shown graphically in Figures 14D, 15A, 16A and 17A

### Multiple copolymer compositions implant:

Using coextrusion, a high-load core PLGA (85:15 PLA:PGA, high IV) copolymer with prolonged release profile is incorporated as the core of composite implant with external low-load, fast release PLGA (50:50 15 PLA:PGA, low IV) which may eliminate the maintenance set and increase degradation time, such that therapeutic levels are sustained for an extensive amount of time allowing for either reimplantation once every 9 months or alternatively reducing implant size.

### Monitoring of blood serum for antipsychotic agent concentration:

2 ml of blood are taken from the subject every three months or as needed based on the implantation/reimplantation regimen. Blood is then centrifuged and the serum separated. Antipsychotic agent concentration is determined following solid-state extraction (MCX, Waters) and HPLC/UV detection by comparison against a known standard.

### Monitoring treatment efficacy:

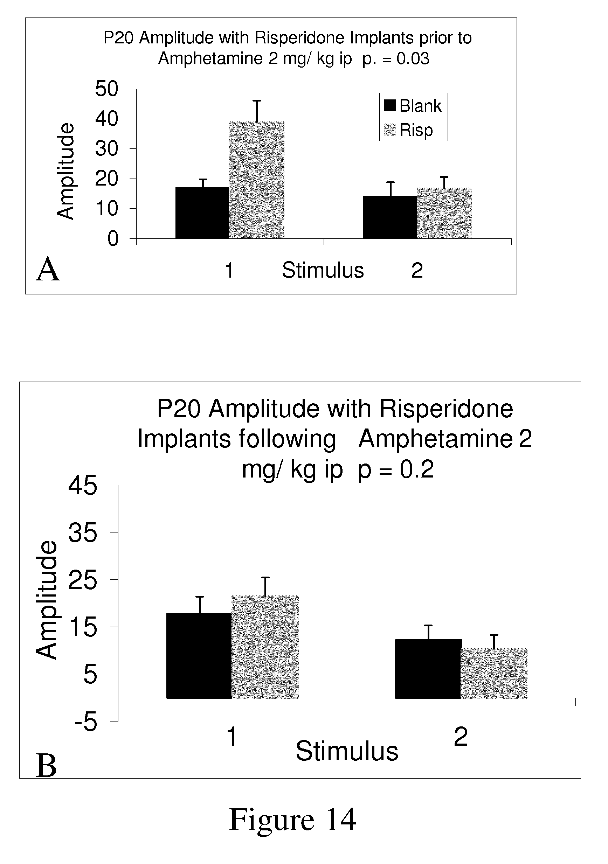

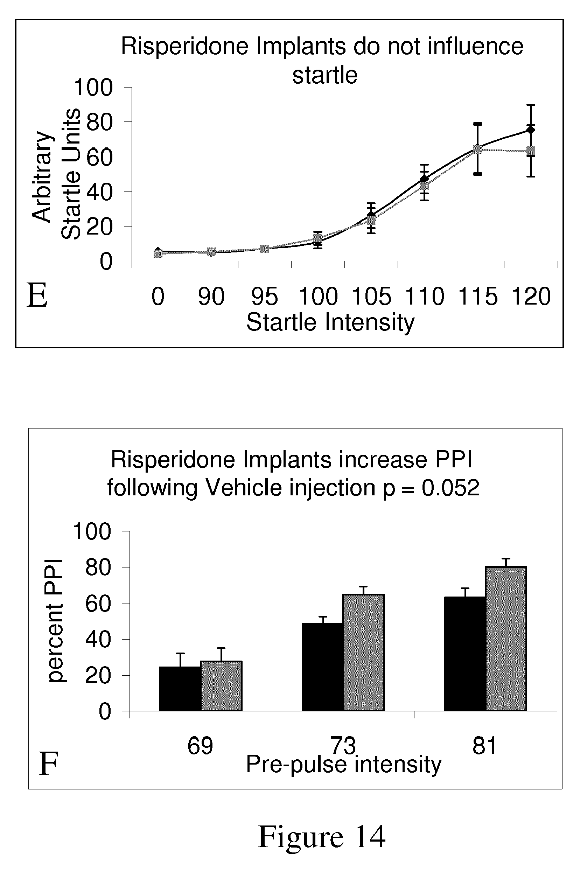

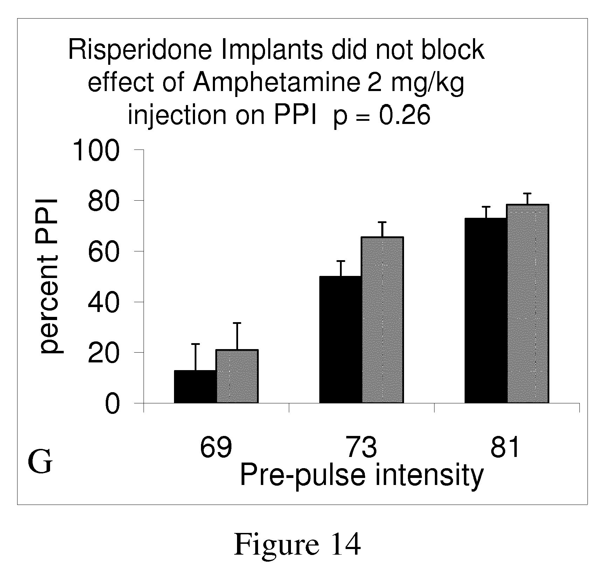

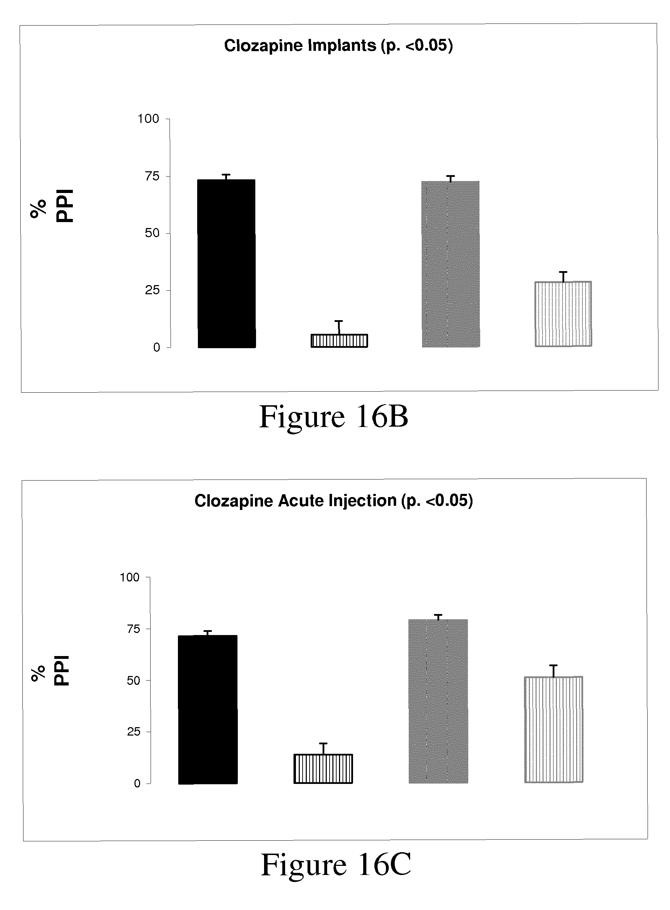

Monitoring efficacy will depend on the psychotic disorder being treated. Patients generally show improvement in cognitive tasks, stable emotion or mood swings, or in particular, improvement in ones ability to work, interact and be intimate with other people and society. No significant side effect are evident and rehospitalization frequency is reduced. Effects of antipsychotic agents released from implants is shown in Figures 14-17.

While various embodiments of the present invention have been described above, it should be understood that they have been presented by way of example only, and not limitation. Thus the breadth and scope of the present invention should not be limited by any of the above described exemplary embodiments, but should be defined only in accordance with the following claims and their equivalents.

### Example 13: Drug loading optimization:

### Background

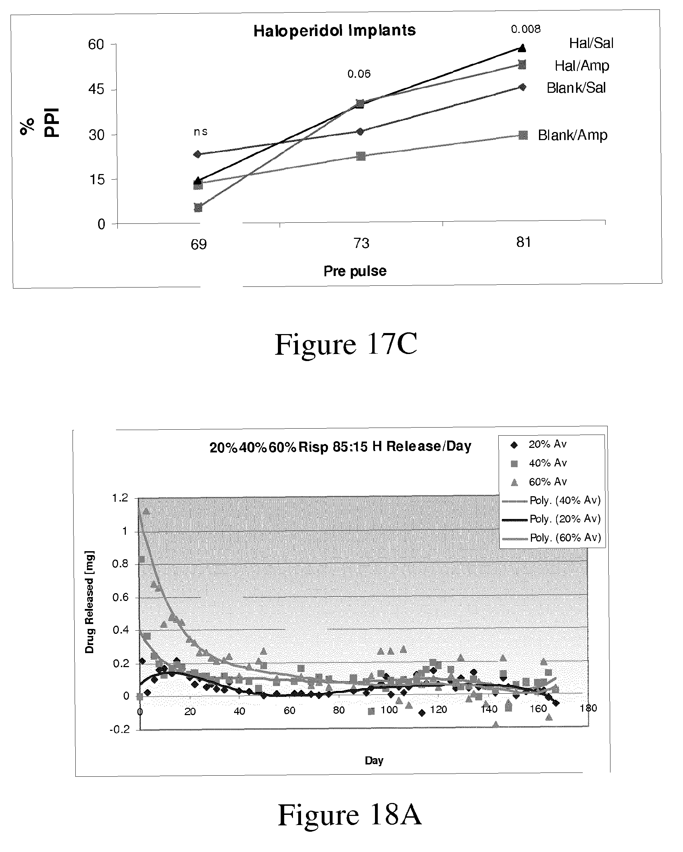

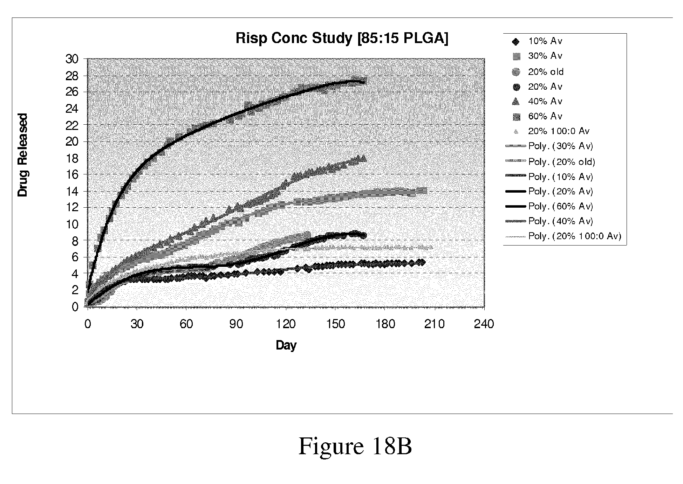

Studies to examine the effects of drug concentration on risperidone release profile were done. The purpose of these studies was to establish the maximum concentration of risperidone that can be used in implants. Implants will utilize the maximum concentration so as to make implants as small as possible and therefore more easily tolerated. Implants were prepared using a single polymer (85:15 PLGA High IV) combined with risperidone at ratios of 20%, 40% or 60% drug by weight (Figure 18A). This polymer has an anticipated degradation interval of 4 months. Each implant has a mass of approximately 50 mg, yielding drug mass of 10, 20 and 30 mg respectively for the 20%, 40% and 60% drug load conditions. These studies were replicated and additional percentages were tested.

### Results

**Figures 18 A-B** shows cumulative risperidone release from implants containing 85:15 PLGA with 20, 40 or 60% drug load by weight. Each point represents the mean of three replicates with a trendline to demonstrate overall pattern of release. **A)** Release expressed in total mg of risperidone from each 50 mg implant. **B)** Release expressed as a percentage of the amount of drug in each type of implant.

Embodiments of the invention include:
1. A method of treating a Nervous System disorder in a subject in need, the method comprising:
   a. Administering to said subject a first formulation of a drug in complex with a biodegradable polymer, wherein said formulation delivers said drug with pseudo-zero order kinetics *in-vivo*; and
   b. Administering to said subject a second formulation of said drug in complex with a biodegradable polymer, wherein said formulation delivers said drug substantially faster, *in-vivo,* than said first formulation,
   whereby administering said first and second formulation results in therapeutic circulating levels of said drug, for a period of about 14-420 days, thereby being a method of treating a nervous system disorder.
2. The method of embodiment 1, wherein said therapeutic circulating levels of said drug range from 0.1 - 10 ng/mL.
3. The method of embodiment 2, wherein administering said second formulation results in said circulating levels within a period of about 1-30 days.
4. The method of embodiment 2, wherein administering said first formulation results in said circulating levels within a period of about 21-180 days
5. The method of embodiment 2, wherein said circulating levels is sustained for about 14-420 days .
6. The method of embodiment 2, wherein the formulations are in a form of an implant.
7. The method of embodiment 6, wherein said implant is inserted subcutaneously.
8. The method of embodiment 1, wherein said first formulation comprises complexes of said drug and two or more biodegradable polymers.
9. The method of embodiment 8, wherein said complexes vary in terms of drug concentration, polymer composition, or combination thereof
10. The method of embodiment 1, wherein said second formulation comprises complexes of said drug and two or more biodegradable polymers.
11. The method of embodiment 10, wherein said complexes vary in terms of drug concentration, polymer composition, or combination thereof
12. The method of embodiment 1, wherein said disorder is AIDS-related dementia, schizophrenia, bipolar disorder, borderline personality disorder (BPD), Alzheimer's disease (AD), psychotic depression or other mental disorders causing confusion, disorganization or psychosis.
13. The method of embodiment 6, wherein said implant is disk or rod shaped.
14. The method of embodiment 13, wherein said rod shaped implant has a diameter of about 1 to about 2 mm, a length of between about 10 and about 40 mm, or a combination thereof.
15. The method of embodiment 1, wherein said drug is at a concentration of between about 2 to about 50 percent by weight of said first or second formulation.
16. The method of embodiment 2, wherein said drug is is Risperidone, 9-OH-Risperidone, haloperidol, olanzapine, clozapine, aripiprazole, quetiapine, ziprasidone or a combination thereof.
17. The method of embodiment 2, wherein said first formulation and said second formulation are administered within 1-24 hours of each other.
18. The method of embodiment 2, wherein said first and second formulations are administered to said subject cyclically.
19. The method of embodiment 18, wherein said first and second formulations are administered to said subject when said circulating levels of said drug serum levels are below 1 ng/mL.
20. The method of embodiment 18, wherein said first and second formulations are administered to said subject from about 160-200 days, following a first administration of the formulations.
21. A kit for sustained delivery of a drug comprising: a first formulation of said drug in complex with a biodegradable polymer, wherein said formulation delivers said drug with pseudo-zero order kinetics in-vivo, and a second formulation of said drug in complex with a biodegradable polymer wherein said second formulation has a rate of release which is faster than said first formulation, in-vivo.
22. The kit of embodiment 21, wherein the combination of said first and second formulation of the kit gives sustained delivery, once administered over a period of about 1 week to about 14 months.
23. The kit of embodiment 21, wherein said biodegradable polymer is copolymer of poly(lactide-glycolide) (PLGA).
24. The kit of embodiment 23, wherein said PLGA is an atactic or syndiotactic block copolymer of PLA and PGA.
25. The kit of embodiment 23, wherein said PLGA has a molecular weight of from about 10,000 to about 200,000.
26. The kit of embodiment 23, wherein the concentration of d,l-lactide monomer comprising said PLGA ranges from 50% - 100%.
27. The kit of embodiment 21, wherein said biodegradable polymer is polylactide.
28. The kit of embodiment 21, wherein said drug is Risperidone, 9-OH-Risperidone, Haloperidol, Olanzapine, Clozapine, Quetiapine, or a combination thereof.
29. The kit of embodiment 21, wherein the drug content is between 2 and 75% (w/w)
30. The kit of embodiment 21, wherein said first formulation is in the form of an implant.
31. The kit of embodiment 30, wherein said implant is for subcutaneous insertion.
32. The kit of embodiment 30, wherein said implant is rod or disk shaped.
33. The kit of embodiment 32, wherein said rod-shaped disk has a diameter of about 1 to about 2 mm, a length of between about 10 and about 40 mm, or a combination thereof.
34. Use of the kit of embodiment 21 in treating a psychotic disorders in a subject in need thereof.
35. A composition for use in the treatment of psychotic disorders, comprising a poly(lactide/glycolide) (PLGA) copolymer at a concentration of from about 95-98% (w/w), and an antipsychotic agent, at a concentration of from about 2 to about 5% (w/w), wherein the lactide:glycolide ratio of said poly(lactide/glycolide) copolymer is from about 100:0 to 50:50 and wherein said antipsychotic agent is Risperidone or 9-OH-Risperidone.
36. The composition of embodiment 35, wherein said PLGA copolymer concentration is 98% (w/w).
37. The composition of embodiment 35, wherein said PLGA copolymer concentration is 95% (w/w).
38. The composition of embodiment 35, wherein said antipsychotic is 9-OH-Risperidone.
39. The composition of embodiment 38, wherein said PLGA has a molar ratio of lactic monomer to glycolic monomer is 85:15.
40. The composition of embodiment 38, wherein said PLGA has a molar ratio of lactic monomer to glycolic monomer is 75:25.
41. The composition of embodiment 38, wherein said PLGA has a molar ratio of lactic monomer to glycolic monomer is 65:35.
42. The composition of embodiment 38, wherein said PLGA has a molar ratio of lactic monomer to glycolic monomer is 50:50.
43. The composition of embodiment 38, in the form of a solid implant.
44. The composition of embodiment 43, wherein said implant is in a disk or rod shape.
45. The composition of embodiment 44, wherein said rod shaped implant has a diameter of about 1 to about 4 mm, a length of between about 10 and about 40 mm, or a combination thereof.

## Claims

1. An implant composition for use in the treatment of psychotic disorders, comprising a poly(lactide/glycolide) (PLGA) copolymer at a concentration of from 40-90% (w/w), and an antipsychotic agent, at a concentration of 10-60% (w/w), wherein the lactide:glycolide molar ratio of said poly(lactide/glycolide) copolymer is from 90:10 to 50:50, and wherein said antipsychotic agent is Risperidone or 9-OH-Risperidone, and wherein said implant composition is a solid implant composition.

2. The implant composition of claim 1, wherein said PLGA copolymer concentration is 50% (w/w).

3. The implant composition of claim 1, wherein said PLGA copolymer concentration is 60% (w/w).

4. The implant composition of claim 1, wherein said antipsychotic agent concentration is 50% (w/w).

5. The implant composition of claim 1, wherein said antipsychotic agent concentration is 40% (w/w).

6. The implant composition of claim 1, wherein said antipsychotic agent is 9-OH-Risperidone.

7. The implant composition of claim 1, wherein said PLGA has a molar ratio of lactic monomer to glycolic monomer is 85:15.

8. The implant composition of claim 1, wherein said PLGA has a molar ratio of lactic monomer to glycolic monomer is 75:25.

9. The implant composition of claim 1, wherein said PLGA has a molar ratio of lactic monomer to glycolic monomer is 65:35.

10. The implant composition of claim 1, wherein said PLGA has a molar ratio of lactic monomer to glycolic monomer is 50:50.

11. The implant composition of claim 1, wherein said implant is in a disk or rod shape.

12. The implant composition of claim 11, wherein said rod shaped implant has a diameter of 1 to 4 mm, a length of between 10 and 40 mm, or a combination thereof.
